(19) Europäisches Patentamt
European Patent Office
Office européen des brevets

(11) **EP 3 310 399 B1**

(12) **FASCICULE DE BREVET EUROPEEN**

(45) Date de publication et mention
de la délivrance du brevet:
**22.05.2019 Bulletin 2019/21**

(21) Numéro de dépôt: **16736798.6**

(22) Date de dépôt: **15.06.2016**

(51) Int Cl.:
*A61L 2/16* *(2006.01)*  *C11D 1/825* *(2006.01)*

(86) Numéro de dépôt international:
**PCT/EP2016/063790**

(87) Numéro de publication internationale:
**WO 2016/202879 (22.12.2016 Gazette 2016/51)**

(54) **MOUSSE AQUEUSE DÉSINFECTANTE, SON PROCÉDÉ DE PRÉPARATION ET SES UTILISATIONS**

DESINFIZIERENDER WÄSSRIGER SCHAUM, VERFAHREN ZUR HERSTELLUNG DAVON UND VERWENDUNG DAVON

DISINFECTING AQUEOUS FOAM, PROCESS FOR PREPARING SAME AND USE THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.06.2015 FR 1555501**

(43) Date de publication de la demande:
**25.04.2018 Bulletin 2018/17**

(73) Titulaire: **Commissariat à l'Energie Atomique et aux Energies Alternatives**
**75015 Paris (FR)**

(72) Inventeurs:
• **FAURE, Sylvain**
**84210 Venasque (FR)**
• **LE TOQUIN, Esther**
**30400 Villeneuve-les-Avignon (FR)**
• **GAS, Fabienne**
**30200 Saint Laurent de Carnols (FR)**

(74) Mandataire: **Brevalex**
**56, Boulevard de l'Embouchure**
**B.P. 27519**
**31075 Toulouse Cedex 2 (FR)**

(56) Documents cités:
**WO-A2-01/45505**  **FR-A1- 2 841 802**
**FR-A1- 2 912 668**  **FR-A1- 2 980 367**
**US-A1- 2010 111 877**  **US-B1- 6 336 977**

**EP 3 310 399 B1**

**Description**

**DOMAINE TECHNIQUE**

La présente invention concerne la décontamination biologique et notamment le traitement de matériaux et/ou d'installations contaminés par des agents pathogènes tels que des bactéries, des virus, et des champignons. Plus particulièrement, la présente invention s'applique à la décontamination/désinfection de surfaces contaminées par de tels agents pathogènes.

En effet, la présente invention propose une mousse aqueuse, gélifiée ou viscosée, à humidité contrôlée et contenant au moins un agent désinfectant ainsi que son utilisation pour le traitement de surfaces contaminées par des agents pathogènes.

**ÉTAT DE LA TECHNIQUE ANTÉRIEURE**

L'utilisation d'agents biologiques comme armes n'est pas une idée nouvelle. L'histoire démontre qu'une telle utilisation existe depuis plus longtemps que la découverte des microbes. Que ce soit par la contamination de puits par des cadavres infectés ou la distribution de couvertures de varioleux pour disséminer l'infection, l'histoire démontre que l'utilisation d'agents pathogènes comme arme est une notion ancienne. Plus récemment, l'attaque par des lettres piégées avec des spores de charbon, survenue aux Etats-Unis à l'automne 2001, a fait prendre conscience aux opinions publiques européenne et américaine de la réalité de la menace bioterroriste.

Dans l'hypothèse d'un accident ou d'un attentat à caractère biologique, la priorité pour les autorités est de limiter les effets sur la population civile. Cette limitation passe par la décontamination rapide des infrastructures exposées afin d'éviter une propagation des agents et de restituer au plus vite les bâtiments à leur usage sans qu'aucun risque d'exposition ne persiste. Certaines zones contaminées difficiles d'accès, comme, par exemple, les conduits d'aération ou les conduits d'évacuation des eaux usées, doivent être rapidement décontaminées pour éviter toute dissémination des agents pathogènes. Il existe donc un besoin sur le marché de moyens de décontamination de ces zones. Un tel moyen de décontamination doit pouvoir être utilisé par remplissage d'un espace clos ou semi-clos ou encore pulvérisé sur les parois verticales et horizontales. De plus, l'identification n'étant pas toujours possible et la réponse se devant d'être rapide, la solution de décontamination doit, quant à elle, être efficace sur une large gamme d'agents biologiques.

La demande de brevet FR 2 980 367 propose un procédé de décontamination d'une surface susceptible d'être contaminée par un agent pathogène ledit procédé comprenant une 1ère étape mettant en oeuvre une solution comprenant une base faible et présentant un pH supérieur ou égal à 9,5 et inférieur à 13 et éventuellement une 2nde étape durant laquelle une solution comprenant un agent oxydant à une concentration d'au moins 50 ppm est utilisé. En outre, les deux solutions peuvent comprendre au moins un agent chélatant et/ou au moins un détergent.

Il existe plusieurs mousses de décontamination biologique et/ou chimique, utilisées dans le domaine des risques Nucléaires, Radiologiques, Biologiques, Chimiques (NRBC).

Une première mousse de décontamination DF-100 a été développée par Sandia National Laboratories. La formulation de cette solution comprend un tensioactif, un composé réactif à savoir du peroxyde d'hydrogène liquide et de l'eau. Une seconde solution a été développée : DF-200 ou EasyDECON® 200 **[1].** Cette solution est une version améliorée de la DF-100, car elle contient en outre un activateur de blanchiment qu'est le diacétate de glycérol. Ce dernier composant permet d'augmenter la vitesse de la réaction, d'améliorer le rendement de la réaction et d'éliminer le besoin d'ajuster le pH. Cette mousse est polyvalente car elle est efficace pour neutraliser les agents chimiques de guerre comme le sarin, l'ypérite, le *O*-éthyl *S*-[2-(diisopropylamino)éthyl] méthylphosphonothioate (ou VX) et le soman, des toxiques chimiques industriels et des agents biologiques tels que *B. anthracis* et *Y. pestis.* Elle n'est pas corrosive et son utilisation ne crée pas de sous-produits nocifs. Telle que fournie dans la demande de brevet US 2007/0249509 **[2],** la formulation complète de la mousse DF-200 est constituée en pourcentage massique par rapport à la masse totale de la formulation :

- de 1,8% de chlorure de benzalkonium (agent tensioactif cationique) ;
- de 0,5% d'ADOGEN 477™ (hydrotrope cationique) ;
- de 1,1% d'hexylène glycol (solvant) ;
- de 0,4% de 1-dodecanol (acide gras) ;
- de 12% de sorbitol (additif sorbant qui agit comme un agent de séchage pour produire une forme granulée) ;
- de 4,7% d'un mélange de carbonate de potassium et de bicarbonate de potassium, utilisé en tant que base forte ;
- de 1,8% de diacétate de glycérol (activateur de blanchiment hydrosoluble) ;
- de 4,6% de polyéthylène glycol (polymère hydrosoluble notamment utilisé pour augmenter la stabilité de la mousse) ;
- de 7,8% de peroxyde d'hydrogène urée (agent décontaminant) et
- de 65,3% d'eau.

Les performances des solutions DF-100 et DF-200 sur des agents chimiques et biologiques comme, par exemple, *Bacillus globigii* (simulant l'Anthrax), *Bacillus anthracis* et *Yersinia pestis* sont accessibles sur internet **[3]**.

La mousse de décontamination de surface CASCAD™ Surface Decontamination Foam (CASCAD™ SDF), commercialisée par Allen-Vanguard, a pour propriétés de décontaminer des immeubles contaminés par des agents biologiques et chimiques, ainsi que des particules radioactives, et de confiner une explosion. Cette solution a été développée pour décontaminer des immeubles sans endommager les différents matériaux contaminés. Elle est la version améliorée de la solution de décontamination CASCAD™ (pour « Canadian Aqueous System for Chemical/biological Agent Decontamination ») **[4]**. Cette dernière est une solution développée pour décontaminer les bateaux, avions et véhicules en cas de suspicion ou de contamination avérée.

La mousse CASCAD™ SDF a été optimisée pour être utilisée sur une plus longue période de temps et dans des conditions climatiques plus contraignantes. Elle se présente sous forme de poudre qui peut être délivrée, après ajout d'eau, sous forme liquide ou sous forme de mousse suivant une large gamme de matériels de dispersion. Après son application et un temps de contact suffisant, la solution peut être rincée ou déplacée à l'aide de pompes.

La mousse CASCAD™ SDF est produite par mélange et réaction de deux solutions liquides ensemble. Ces dernières sont préparées à partir de trois réactifs séparés qui présentent les compositions chimiques suivantes :

- GPA-2100 (décontaminant) : réactif solide sous forme poudreuse consistant en un sel sodique d'acide dichloroisocyanurique (70 à 100% en masse) ;
- GPA-2100 (tampon) : réactif solide sous forme poudreuse consistant en tétraborate de sodium (10 à 30% en masse), hydroxyde de sodium (1 à 5% en masse) et carbonate de sodium (40 à 65% en masse) ; et
- GCE-2000 (tensioactif) : réactif liquide consistant en sulfate de sodium myristique (10 à 30% en masse), sulfonate d'oléfine (C14-16) de sodium (10 à 30% en masse), éthanol dénaturé (3 à 9% en masse), alcools (C10-16) (5 à 10% en masse), sulfate de sodium (3 à 7% en masse), xylène sulfonate de sodium (1 à 5% en masse) et un mélange de sels de sodium et d'ammonium avec de l'eau et un co-solvant (quantité supérieure à 9% en masse).

Un procédé de préparation de la mousse CASCAD™ SDF est notamment fourni dans l'Annexe B de **[5]**. Ce rapport propose une étude comparative de l'efficacité de décontamination de plusieurs solutions de décontamination dont la DF-200, la CASCAD™ SDF et l'eau de Javel, sur différents matériaux. Cette étude porte sur six solutions de décontamination :

- pH-Amended Bleach utilisée sous forme liquide et composée d'hypochlorite de sodium à 5%, d'eau et de 5% d'acide acétique pour ajuster le pH ;
- CASCAD™ SDF (Allen-Vanguard) utilisée sous forme de mousse ;
- Decon Green utilisée sous forme liquide, l'agent actif étant le peroxyde d'hydrogène ;
- EasyDECON® 200 (DF-200) (EFT Holdings, Inc.) utilisée sous forme liquide ;
- Spor-Klenz® RTU (STERIS Corporation) utilisée sous forme liquide, les agents actifs étant le peroxyde d'hydrogène et l'acide peracétique ;
- Peridox® RTU (CET, LLC) utilisée sous forme liquide, les agents actifs étant le peroxyde d'hydrogène et l'acide peracétique.

Les résultats obtenus quant à l'efficacité de décontamination vis-à-vis des spores de *Bacillus anthracis* et présentés dans **[5]** montrent que les matériaux poreux tels que le béton, l'asphalte et le bois traité, sont plus difficiles à décontaminer que les matériaux non-poreux tels que le verre, l'acier inoxydable, l'aluminium, la porcelaine et le granit. La solution la plus efficace pour décontaminer les spores est la mousse CASCAD™ SDF. Elle est efficace à la fois sur les matériaux poreux et non-poreux. La solution EasyDECON 200 et celle à base d'eau de Javel ne sont pas efficaces sur des matériaux poreux tels que l'asphalte et le bois traité. Pour ces trois solutions, aucun dommage n'a été constaté sur les matériaux après 60 min de temps de contact et 7 jours après le comptage des spores.

La demande de brevet FR 2 912 668 propose une mousse de décontamination, de décapage et/ou de dégraissage stabilisée par des particules solides. La solution moussante peut contenir, en tant qu'agent de décontamination, de décapage et/ou de dégraissage, un agent désinfectant ou un antiseptique. La mousse objet de cette demande présente un foisonnement compris entre 5 et 20 et, en particulier, compris entre 10 et 15.

La demande internationale WO 01/45505 propose un procédé pour éliminer des plantes non désirées mais aussi des bactéries et des virus à l'aide d'une mousse chaude. La solution moussante mise en oeuvre contient des agents tensioactifs anioniques particuliers en une quantité comprise entre 0,1 et 25% en poids mais ne contient aucun agent désinfectant.

En résumé, les mousses de l'art antérieur sont polyvalentes sur les agents chimiques et biologiques. Elles sont utilisées en majorité par pulvérisation sur les surfaces à traiter. A noter cependant que les solutions moussantes à partir desquelles elles sont préparées présentent de nombreux constituants ce qui est non seulement coûteux mais aussi entraîne des procédés de préparation longs. Enfin, le foisonnement de ces mousses est non spécifié et donc non contrôlé.

Les inventeurs se sont fixé pour but de mettre au point une mousse utile pour le traitement des surfaces contaminées par les agents biologiques, facile à mettre en oeuvre et ce, quelle que soit la surface à traiter, ne nécessitant ni structure, ni réactif onéreux et générant que très peu d'effluent une fois le traitement réalisé.

## EXPOSÉ DE L'INVENTION

Les buts fixés et d'autres encore sont atteints par l'invention qui propose une mousse aqueuse à humidité contrôlée et un procédé de traitement et de désinfection des surfaces contaminées.

La présente invention est une mousse aqueuse à humidité contrôlée par un générateur spécifique. Ces propriétés physico-chimiques permettent sa stabilité dans le temps sous forme de mousse grâce à l'utilisation dans la formulation d'un agent viscosant. Le contrôle du foisonnement par le générateur permet d'obtenir des mousses stables et efficaces sur les agents pathogènes avec un pourcentage d'humidité compris entre 2% et 8%, et préférentiellement entre 4% et 5%.

La mise en oeuvre et la récupération de la mousse selon l'invention sont originales. En effet, le foisonnement contrôlé entre 12,5 et 50 et avantageusement entre 20 et 25 permet une utilisation par pulvérisation ou étalement en « couche » (ou talochage) d'une mousse stable et adhérente sur les parois inclinées, horizontales ou verticales, les sols et les plafonds. La mousse selon la présente invention peut être aussi utilisée en remplissage de milieux clos ou semi-clos pouvant être de volume variable et important. La récupération de cette mousse peut se faire par aspiration ou en la laissant simplement s'évaporer, l'évaporation laissant des traces non toxiques.

La présente invention propose donc une mousse évaporable ou aspirable ce qui constitue un concept tout à fait nouveau eu égard aux mousses de l'art antérieur. De plus, en termes d'effluents liquides, une mousse aspirable n'en génère que peu et une mousse évaporable n'en génère aucun.

De par sa composition, la mousse aqueuse désinfectante selon la présente invention dispose des avantages des mousses à durée de vie contrôlée classiquement utilisées dans le traitement de décontamination radioactive (voir, à cet effet, la demande internationale WO 2004/008463 **[6]**). La mousse aqueuse désinfectante selon la présente invention se distingue toutefois des mousses décrites dans la demande internationale WO 2004/008463 de par l'absence d'agent de décontamination radiologique et de par son foisonnement.

A noter que la solution aqueuse moussante mise en oeuvre pour préparer la mousse aqueuse désinfectante selon la présente invention ne contient, en plus de l'eau, que trois types de composés ce qui correspond à une formulation simplifiée vis-à-vis des formulations des mousses de l'état de la technique. Une telle solution aqueuse moussante est donc constituée

- de 0,05% à 1,5% en masse d'un agent tensioactif organique moussant ou d'un mélange d'agents tensioactifs organiques moussants,
- de 0,05% à 0,8% en masse d'un agent gélifiant ou viscosant, organique ou d'un mélange d'agents gélifiants ou viscosants, organiques,
- de 1% à 14% en volume d'un agent désinfectant ou d'un mélange d'agents désinfectants et
- d'eau,
  ladite mousse présentant un foisonnement compris entre 20 et 50.

La solution moussante utilisée pour préparer la mousse aqueuse désinfectante comprend, comme solvant, de l'eau justifiant ainsi l'appellation de solution aqueuse moussante. Par « eau », on entend l'eau de distribution, l'eau désionisée ou encore l'eau distillée. Avantageusement, la mousse aqueuse désinfectante de l'invention peut être une mousse neutre, acide ou basique et ce, en fonction du ou des agent(s) désinfectant(s) qu'elle contient et des conditions de pH requises pour une bonne efficacité désinfectante de ce ou ces dernier(s). L'homme du métier saura déterminer le pH le plus adapté et modifier, en conséquence, le pH de la solution aqueuse moussante.

La mousse aqueuse désinfectante de l'invention est une mousse à foisonnement contrôlé et donc à une humidité contrôlée. Pour rappel, une mousse est souvent caractérisée par son foisonnement défini, dans les conditions normales de température et de pression, par la relation (I) suivante :

$$F = (Vol_{gaz} + Vol_{liquide})/Vol_{liquide} = Vol_{mousse}/Vol_{liquide} \quad (I)$$

Par conséquent, l'humidité d'une mousse correspond à l'inverse de son foisonnement et donc est définie par le rapport $Vol_{liquide}/Vol_{mousse}$.

Les mousses aqueuses désinfectantes de la présente invention présentent un foisonnement compris entre 20 et 50, notamment entre 20 et 30 et, en particulier, entre 20 et 25, ce qui correspond à une fraction liquide ou humidité de la mousse comprise entre 2 et 8%, notamment entre 3,33 et 6,67% et, en particulier, entre 4 et 5%. A noter que le volume de liquide (Vol$_{liquide}$) dans les rapports ci-dessus correspond aux volumes des différents composés mélangés initialement pour préparer la solution aqueuse moussante et, en particulier, à la somme du volume du ou des agent(s) tensioactif(s) organique(s) moussant(s), du volume du ou des agent(s) gélifiant(s) ou viscosant(s), organique(s), du ou des agent(s) désinfectant(s) et du volume d'eau.

La solution aqueuse moussante générant la mousse aqueuse désinfectante de l'invention comprend au moins un agent tensioactif organique moussant. Par « tensioactif organique », on entend une molécule organique comportant une partie lipophile (apolaire) et une partie hydrophile (polaire). Par « tensioactif organique moussant », on entend un tensioactif organique tel que précédemment défini présentant en outre un équilibre hydrophile/lipophile (ou HLB pour « Hydrophilic-Lipophilic Balance ») compris entre 3 et 8. Pour rappel, la valeur HLB d'un tensioactif peut facilement être obtenue grâce à la formule de Davies [7] et aux tables de HLB pour différents groupes chimiques, disponibles pour l'homme du métier.

Plus particulièrement, la solution aqueuse moussante constituant la mousse aqueuse désinfectante de l'invention peut comprendre un seul agent tensioactif organique moussant ou un mélange d'au moins deux agents tensioactifs organiques moussants choisi(s) parmi les tensioactifs moussants non ioniques, les tensioactifs moussants anioniques, les tensioactifs moussants cationiques, les tensioactifs amphotères, les tensioactifs de structure de type Bolaforme, les tensioactifs de structure de type Gemini et les surfactants polymériques.

Avantageusement, la solution aqueuse moussante mise en oeuvre dans le cadre de la présente invention comprend un seul agent tensioactif organique moussant ou un mélange d'au moins deux agents tensioactifs organiques moussants choisi(s) parmi les tensioactifs moussants non ioniques, les tensioactifs moussants anioniques et les tensioactifs moussants cationiques. Dans les mélanges de tensioactifs organiques moussants, au moins deux tensioactifs sont choisis dans la même famille ou dans deux familles différentes choisie(s) parmi les tensioactifs moussants non ioniques, les tensioactifs moussants anioniques et les tensioactifs moussants cationiques.

Pour rappel, les tensioactifs non-ioniques (ou neutres) sont des composés dont les propriétés tensioactives, notamment l'hydrophilie, sont apportées par des groupements fonctionnels non chargés tels qu'un alcool, un éther, un ester ou encore un amide, et peuvent contenir des hétéroatomes tels que l'azote ou l'oxygène. En raison de la faible contribution hydrophile de ces fonctions, les composés tensioactifs non-ioniques sont le plus souvent polyfonctionnels. Dans le cadre de la présente invention, les tensioactifs non-ioniques moussants sont notamment choisis parmi les alcoxylates d'alkyles ; les alcoxylates d'alcools gras ; les alcoxylates d'amines grasses ; les alcoxylates d'acides gras ; les alcoxylates d'oxoalcools ; les alcoxylates d'alkylphénols ; les éthoxylates d'alkyles ; les éthoxylates d'alcools gras; les éthoxylates d'amines grasses; les éthoxylates d'acides gras; les éthoxylates d'oxoalcools ; les éthoxylates d'alkylphénols comme, par exemple, les éthoxylates d'octylphénol et de nonylphénol ; les alcools, les α-diols, les alkylphénols polyéthoxylés et poly-propoxylés ayant une chaîne grasse comportant, par exemple, de 8 à 18 atomes de carbone, le nombre de groupes oxydes d'éthylène ou oxydes de propylène pouvant être notamment de 2 à 50 ; les polymères complexes d'oxydes de polyéthylène et de polypropylène ; les copolymères d'oxyde d'éthylène et de propylène ; les copolymères blocs d'oxydes de polyéthylène et de polypropylène comme, par exemple, les copolymères triblocs POE-POP-POE ; les condensats d'oxyde d'éthylène et de propylène sur des alcools gras; les amides gras polyéthoxylés ayant, de préférence, de 2 à 30 moles d'oxyde d'éthylène ; les éthers polyéthoxylés ayant, de préférence, de 2 à 30 moles d'oxyde d'éthylène ; les monoesters (monolaurate, monomyristate, monostéarate, monopalmitate, monooléate, etc) et polyesters d'acides gras et du glycérol ; les amides gras polyglycérolés comportant en moyenne de 1 à 5 et, plus spécialement, de 1,5 à 4 groupes glycérol ; les esters d'acide gras du sorbitan oxyéthylénés comportant de 2 à 30 moles d'oxyde d'éthylène ; les monoesters (monolaurate, monomyristate, monostéarate, monopalmitate, monooléate, etc) et polyesters d'acides gras et du sorbitane, les monoesters de polyoxyéthylène sorbitane ; les esters d'acides gras du sucrose ; les esters d'acides gras du polyéthylèneglycol ; les alkylpolyglucosides ; les dérivés de N-alkyl glucamine et les oxydes d'amines tels que les oxydes d'alkyl(C$_{10}$-C$_{14}$) amines ou les oxydes de N-acylaminopropylmorpholine ; les polyols (tensioactifs dérivés de sucres) en particulier les alkylates de glucose tels que par exemple l'hexanate de glucose ; les tensioactifs dérivant de glucoside (laurate de sorbitol) ou de polyols tels que les éthers d'alcools glycérolés ; les alcanolamides et leurs mélanges. Plus particulièrement, à titre de tensioactifs non-ioniques moussants, il est possible d'employer les tensioactifs non ioniques moussants décrits dans la demande internationale WO 2004/008463 [6]. Un tel tensioactif est, par exemple, choisi dans la famille des alkylpolyglucosides ou des alkylpolyétherglucosides, dérivés naturels du glucose et biodégradables. Ce sont par exemple l'« ORAMIX CG-110 » de la société SEPPIC, ou encore le « Glucopon 215 CS » de la société COGNIS.

Les tensioactifs anioniques sont des tensioactifs dont la partie hydrophile est chargée négativement. Un agent tensioactif anionique moussant utilisable dans le cadre de la présente invention est typiquement choisi dans le groupe constitué par les esters d'acide sulfurique, les esters d'acide phosphorique, les alkyle ou aryle sulfonates, les alkyle ou aryle sulfates, les alkyle ou aryle phosphates, les alkyle ou aryle sulfosuccinates ou les alkyle ou aryle sarcosinates associés à un contre ion comme un ion ammonium (NH$_4^+$), un ammonium quaternaire tel que tétrabutylammonium, et les cations et notamment les cations alcalins, lesdits cations étant tels que Na$^+$, Li$^+$, Ca$^{2+}$, Mg$^{2+}$, Zn$^{2+}$ et K$^+$. A titre de tensioactifs anioniques moussants, il est, par exemple, possible d'utiliser le paratoluènesulfonate de tetraéthylammonium, le dodécylsulfate de sodium (ou SDS), le laurylsarcosinate de sodium (ou sarcosyl), le palmitate de sodium, le stéarate de sodium, le myristate de sodium, le di(2-éthylhexyl) sulfosuccinate de sodium, le méthylbenzène sulfonate et l'éthylbenzène sulfonate.

Les agents tensioactifs cationiques présentent au moins une chaîne hydrocarbonée et une tête polaire, la partie hydrophile dudit agent étant chargée positivement. Un agent tensioactif cationique moussant utilisable dans le cadre de la présente invention est avantageusement choisi parmi les ammoniums quaternaires comportant au moins une chaîne aliphatique en $C_4$-$C_{22}$ associés à un contre-ion anionique choisi notamment parmi les dérivés du bore tels que le tétrafluoroborate ou les ions halogénures tels que F-, Br-, I- ou Cl-. A titre d'agents tensioactifs cationiques moussants utilisables, on peut citer le chlorure tétrabutylammonium, le chlorure tétradécylammonium, le bromure de tétradécyltriméthyle ammonium (TTAB), les halogénures d'alkylpyridinium portant une chaîne aliphatique et les halogénures d'alkylammonium.

Dans un mode de réalisation particulier, le ou les tensioactif(s) organique(s) moussant(s) est/sont choisi(s) dans le groupe constitué par les sels d'acide carboxylique, les sels d'acide sulfonique, les sels de sulfate, les sels d'esters d'acide sulfurique, les sels d'esters de l'acide phosphorique, les alkylpolyglucosides et les oxydes d'amines.

Dans la solution aqueuse moussante constituant la mousse aqueuse désinfectante de la présente invention, l'agent tensioactif ou le mélange d'au moins deux agents tensioactifs est présent à raison de 0,05 à 1,5% en masse, notamment de 0,08 à 1,3% en masse et, en particulier, de 0,1 à 1,1% en masse.

De plus, la solution aqueuse moussante constituant la mousse aqueuse désinfectante de la présente invention comprend, en plus du (ou des) agent(s) tensioactif(s) précédemment cité(s), un agent gélifiant ou viscosant, organique ou un mélange d'au moins deux agents gélifiants ou viscosants, organiques dans une teneur comprise entre 0,05% et 0,8% en masse, notamment de 0,1 à 0,5% en masse et, en particulier, de 0,15 à 0,3% en masse.

Avantageusement, un tel agent gélifiant organique est un agent biodégradable et pseudo-plastique permettant à la mousse d'être aisément pulvérisable et de présenter une durée de vie comprise entre 30 min et 6 h et donc adaptée à la durée de décontamination biologique et d'utilisation.

Ce ou ces agent(s) gélifiant(s) ou viscosant(s) est/sont, plus particulièrement, choisi(s) parmi les polymères hydrosolubles, les hydrocolloïdes, les hétéropolysaccharides tels que, par exemple, les polymères polyglucosidiques à chaînes ramifiées trisaccharidiques, les dérivés cellulosiques et les polysaccharides tels que les polysaccharides contenant du glucose comme seul monomère. A titre d'exemples particuliers, le ou les agent(s) gélifiant(s) ou viscosant(s) utilisables dans le cadre de la présente invention est/sont choisi(s) dans le groupe constitué par la gomme xanthane, la gomme de guar, l'agar-agar, le carraghénane, l'alginate de sodium, le caséinate, la gélatine, la pectine, l'amidon, la cellulose, la 2-hydroxyéthylcellulose (HEC) et le chitosan.

Enfin, la solution aqueuse moussante constituant la mousse aqueuse désinfectante de la présente invention comprend, en plus du ou des agent(s) tensioactif(s) organique(s) moussant(s) et du ou des agent(s) gélifiant(s) ou viscosant(s), organique(s) précédemment cité(s), un agent désinfectant ou un mélange d'au moins deux agents désinfectants dans une teneur comprise entre 1% et 14% en volume.

L'agent désinfectant ou le mélange d'agents désinfectants peut être présent dans la solution aqueuse moussante en une quantité comprise entre 1 et 10% en volume, notamment entre 2 et 7,5% en volume et, en particulier, de l'ordre de 5% (i.e. 5% ± 1%) en volume. Ces gammes particulières sont notamment mises en oeuvre pour des surfaces qui ne sont ni en métal, ni en acier et pour lesquelles aucune réaction avec le ou les agent(s) désinfectant(s) n'a été montrée.

En variante et notamment pour des surfaces métalliques ou en acier, la teneur en agent désinfectant ou en mélange d'au moins deux agents désinfectants est typiquement comprise entre 5% et 14% en volume et, en particulier, de l'ordre de 12% (i.e. 12% ± 1%) en volume. Dans ce cas de figure, le ou les agent(s) désinfectant(s) peuvent réagir avec de telles surfaces comme les surfaces en aluminium.

Le ou les agent(s) désinfectant(s) utilisable(s) dans le cadre de la présente invention appartien(nen)t aux produits biocides tels que définis par la règlementation concernant la mise à disposition sur le marché et l'utilisation des produits biocides (Règlement UE n°528/2012 du 22 mai 2012 **[8]**). Ces produits biocides représentent l'ensemble des substances et mélanges, constitués d'une ou de plusieurs molécule(s) active(s), destinés à détruire, repousser ou rendre inoffensifs les organismes nuisibles vivants, à en prévenir l'action ou à les combattre de toute autre manière par une action chimique ou biologique. Ces produits sont divisés, suivant leurs applications, en quatre groupes qui sont (i) les agents désinfectants, (ii) les produits de protection visant à prévenir le développement microbien et le développement des algues, (iii) les produits de lutte contre les nuisibles et (iv) les autres produits biocides comme les produits antisalissures ou pour l'embaumement et la taxidermie.

Les agents désinfectants sont des produits ou procédés utilisés pour la désinfection ou la décontamination de matériaux contaminés et sont applicables sur des surfaces inertes, tissus vivants ou denrées alimentaires. Ainsi, les agents désinfectants servent à traiter notamment les dispositifs médicaux, les sols et les surfaces tels que le métal, le béton, la brique, la céramique, le bois et le plastique qui sont des matériaux utilisés dans les infrastructures critiques ainsi que le matériel sensible.

L'efficacité des agents désinfectants est dépendante de leur spectre d'action sur les différents types d'agents biologiques. Ainsi, sont définis des agents bactéricides (action sur les bactéries), des agents fongicides (action sur les champignons), des agents virucides (action sur les virus) et des agents sporicides (action sur les spores). De plus, chaque agent désinfectant présente plusieurs critères de performance, comme (i') sa vitesse d'efficacité, (ii') son efficacité de décontamination qui se mesure par un facteur de diminution d'une population contaminante initiale sous l'effet du désinfectant (population initiale/population finale après traitement) ou par la réduction en $\log_{10}$ de ce facteur et (iii') sa compatibilité avec les matériaux de construction. Les agents désinfectants sont donc classés en fonction de leur efficacité de désinfection et on parle d'agents désinfectants à niveau de désinfection haut, moyen ou bas.

Dans le cadre de la présente invention, le ou les agent(s) désinfectant(s) mis en oeuvre est/sont choisi(s) parmi les agents désinfectants à niveau de désinfection haut i.e. les agents désinfectants présentant un facteur (population contaminante initiale/population finale après traitement) supérieur à $10^6$. Avantageusement, ces facteurs sont choisis parmi les produits chlorés, les aldéhydes et les oxydants.

Les produits chlorés sont des agents désinfectants à spectre d'activité étendu puisqu'ils sont bactéricides, virucides, fongicides et sporicides. Leur temps d'action est rapide et égal à leur temps de séchage. Ils sont cependant soumis à des facteurs influençant leur activité tels que le pH et la température. De plus, leur activité est inhibée en présence d'ions de métaux lourds, d'un biofilm, de matière organique dissoute, à basse température, à pH faible, ou en présence d'un rayonnement UV. Ils sont utilisés comme désinfectants de surfaces, d'effluents liquides et de matériels.

A titre d'exemples de produits chlorés utilisables comme agent désinfectant dans le cadre de la présente invention, on peut citer le chlore, l'hypochlorite de sodium (eau de Javel) et le dioxyde de chlore. A noter que le pH de l'hypochlorite de sodium qui est en moyenne de 11 peut être ajusté pour qu'il soit compris entre 5 et 8. En effet, à ce pH, l'hypochlorite de sodium est plus efficace comme désinfectant et devient probablement moins agressif pour les matériaux.

Les aldéhydes ont un spectre d'activité étendu car ils sont bactéricides, fongicides, virucides et sporicides. Ils sont utilisés sous forme liquide ou gazeuse pour désinfecter les surfaces, les équipements, les locaux et les dispositifs médicaux. Ils ont comme action de provoquer une dénaturation des acides nucléiques et des protéines des microorganismes.

A titre d'exemples d'aldéhydes utilisables comme agent désinfectant dans le cadre de la présente invention, on peut citer le glutaraldéhyde et l'aldéhyde succinique.

Les oxydants ont un spectre d'activité étendu car ils sont bactéricides, virucides, fongicides et sporicides. Leur efficacité est meilleure en pH acide et ils sont inhibés par la présence de matières organiques. Ils ont pour action de détruire les membranes organiques. Ils sont utilisés surtout sous forme vapeur pour la désinfection des surfaces et du matériel.

A titre d'exemples d'oxydants utilisables comme agent désinfectant dans le cadre de la présente invention, on peut citer les peroxydes comme le peroxyde d'hydrogène ; les peroxydes activés comme le peroxyde d'hydrogène + bicarbonate, le peroxyde d'hydrogène + urée, le peroxyde d'hydrogène + acide peracétique et le peroxyde d'hydrogène + fer (réactif de Fenton) ; les hydroperoxycarbonates ; l'acide peracétique ; le perborate sodique ; le percarbonate sodique éventuellement perhydraté ; le peroxysilicate sodique ; le peroxypyrophosphate sodique ; le peroxysilicate sodique et les aryloxydes comme les arylbenzènesulfonates.

Dans un mode de réalisation particulier de l'invention, le ou les agent(s) désinfectant(s) moussant(s) est/sont choisi(s) dans le groupe constitué par les produits chlorés et les oxydants. Plus particulièrement encore, le ou les agent(s) désinfectant(s) moussant(s) est/sont choisi(s) dans le groupe constitué par l'hypochlorite de sodium et le peroxyde d'hydrogène.

Le gaz mis en oeuvre pour générer la mousse aqueuse désinfectante selon l'invention peut être un quelconque gaz. Il peut notamment être choisi dans le groupe constitué par l'air, l'oxygène, le dioxyde de carbone, l'hélium, l'argon et l'azote. Avantageusement, le gaz utilisé dans le cadre de la présente invention est de l'air. Ainsi, la mousse aqueuse désinfectante selon l'invention consiste en une dispersion de bulles d'air dans une solution moussante telle que précédemment définie.

A titre d'exemples particuliers de mousse aqueuse désinfectante selon l'invention, on peut citer :

(1) une dispersion de bulles de gaz et notamment d'air dans une solution moussante constituée :

- de 0,1 à 1,1% en masse d'un agent tensioactif organique moussant ou d'un mélange d'agents tensioactifs organiques moussants,
- de 0,15% à 0,3% en masse d'un agent gélifiant ou viscosant, organique ou d'un mélange d'agents gélifiants ou viscosants, organiques,
- de 2% à 7,5% en volume et notamment de l'ordre de 5% en volume d'un agent désinfectant ou d'un mélange d'agents désinfectants et

- de l'eau (ou d'eau) ;

(2) une dispersion de bulles de gaz et notamment d'air dans une solution moussante comprenant constituée :

- de 0,1 à 1,1% en masse d'un agent tensioactif organique moussant ou d'un mélange d'agents tensioactifs organiques moussants,
- de 0,15% à 0,3% en masse d'un agent gélifiant ou viscosant, organique ou d'un mélange d'agents gélifiants ou viscosants, organiques,
- de 5% à 14% en volume et notamment de l'ordre de 12% en volume d'un agent désinfectant ou d'un mélange d'agents désinfectants et
- de l'eau (ou d'eau) ;

(3) une dispersion de bulles de gaz et notamment d'air dans une solution moussante comprenant constituée :

- de 0,1 à 1,1% en masse d'un alkylpolyglucoside,
- de 0,15% à 0,3% en masse de gomme de xanthane,
- de 2% à 7,5% en volume et notamment de l'ordre de 5% en volume d'hypochlorite de sodium ou de peroxyde d'hydrogène et
- de l'eau (ou d'eau) ;

(4) une dispersion de bulles de gaz et notamment d'air dans une solution moussante comprenant constituée :

- de 0,1 à 1,1% en masse d'un alkylpolyglucoside,
- de 0,15% à 0,3% en masse de gomme de xanthane,
- de 5% à 14% en volume et notamment de l'ordre de 12% en volume d'hypochlorite de sodium ou de peroxyde d'hydrogène et
- de l'eau (ou d'eau) ;
le foisonnement de ces mousses étant tel que précédemment défini.

La présente invention propose également un procédé pour préparer la mousse aqueuse désinfectante telle que précédemment définie. Cette dernière peut être aisément préparée, à température ambiante (i.e. à une température de l'ordre de 23°C $\pm$ 5°C), par des techniques connues de l'homme du métier.

La première étape de ce procédé de préparation consiste à mélanger ensemble l'eau, le ou les agent(s) tensioactif(s) organique(s) moussant(s), le ou les agent(s) gélifiant(s) ou viscosant(s), organique(s) et le ou les agent(s) désinfectant(s), avant génération de la mousse. Ce mélange peut être effectué par ajout des composants en une seule fois, par groupe ou les uns après les autres. Dans une forme de mise en oeuvre particulière, il peut être envisagé de préparer une 1ère solution en mélangeant ensemble l'eau, le ou les agent(s) tensioactif(s) organique(s) moussant(s) et le ou les agent(s) gélifiant(s) ou viscosant(s), organique(s) et de n'ajouter à cette solution le ou les agent(s) désinfectant(s) que juste avant de générer la mousse.

La seconde étape de ce procédé de préparation consiste à générer la mousse. Cette étape peut être réalisée par tout système de génération de mousse de l'art antérieur et connu de l'homme du métier. Il s'agit de tout dispositif assurant le mélange gaz-liquide, notamment par agitation mécanique, par barbotage, par mélangeur statique contenant des billes ou non, par générateur de mousse à tube à microbilles ou encore des dispositifs décrits dans la demande internationale WO 02/043847 [9], ou tout autre dispositif notamment les systèmes à buses ou venturi permettant des débits importants généralement compris entre 1 et 1000 m$^3$/h. Plus particulièrement l'invention trouve son intérêt dans l'utilisation d'un générateur de mousse qui permet de maîtriser l'humidité de la mousse générée. Ce contrôle de l'humidité s'effectue en mesurant le débit de solution et d'air mélangé. Les formulations de l'invention permettent aisément d'obtenir une mousse avec ce dernier type de générateur dont l'humidité est comprise entre 2 et 5%, notamment entre 3,33 et 5% et, en particulier, entre 4 et 5%.

La présente invention concerne l'utilisation d'une mousse aqueuse désinfectante telle que précédemment définie pour traiter une surface susceptible d'être contaminée par au moins un agent biologique. Plus particulièrement, la présente invention concerne un procédé pour traiter une surface susceptible d'être contaminée par au moins un agent biologique consistant à mettre en contact ladite surface avec une mousse aqueuse désinfectante.

Par « traiter une surface susceptible d'être contaminée par au moins un agent biologique », on entend dans le cadre de la présente invention diminuer la quantité d'agents biologiques présents sur la surface avant le traitement selon l'invention. Cette diminution peut impliquer l'élimination ou la destruction de ces agents et/ou leur transformation en éléments moins nocifs. Ainsi, l'expression « traitement d'une surface » est équivalente et interchangeable avec les expressions « désinfection d'une surface » et « décontamination biologique d'une surface ».

Toute surface susceptible d'être contaminée par un ou plusieurs agent(s) biologique(s) peut être soumise à un procédé de traitement selon la présente invention. Par « surface » il faut entendre la partie extérieure d'un objet ou corps solide, qui le limite en tout sens. Il est possible, pour un même objet (ou même corps solide), de définir conceptuellement différentes surfaces. L'invention s'applique à tout type de surface quelle que soit sa géométrie. Cette dernière peut être simple, comme une surface parfaitement plane, ou complexe, comme une surface rugueuse, ou présentant des cavités non obstruées et ce quel que soit le matériau constituant la surface et le reste de l'objet dont elle dépend.

Avantageusement, dans le cadre de la présente invention, la surface de l'objet à traiter peut être une surface inorganique ou organique et notamment une surface en métal comme en aluminium, en alliage métallique, en acier et notamment en acier inoxydable, en fer-blanc, en silicium, en verre contenant généralement des silicates, en verre de silice, en céramique, en brique, en porcelaine, en ciment, en béton, en asphalte, en pierre, en granit, en bois, en terre, en plastique ou en une quelconque de leurs associations.

La surface à traiter ou l'objet dont on souhaite traiter la surface conformément au procédé de la présente invention peut présenter une taille, une forme et une orientation quelconques. Il peut s'agir de grandes surfaces telles qu'une route ou le mur, le plafond et/ou le sol d'une grande infrastructure comme un immeuble, un aéroport, un métro ou un hôtel, des installations de taille intermédiaire telles qu'un objet industriel comme une machine utilisée dans l'agro-alimentaire, un véhicule, une carcasse, un aéronef, une cuve, une cuisine de restaurant, une chambre froide, un sanitaire ou un conteneur et des installations de petite taille comme des dispositifs médicaux, des tuyaux, ou encore une arme.

Par « agent biologique », on entend les micro-organismes naturels tels que bactéries, archées, parasites, protozoaires, champignons, levures ou virus, les toxines produites ou non par de tels micro-organismes, les agents pathogènes de nature protéique comme les prions et les micro-organismes génétiquement modifiés.

A titre d'exemples particuliers et non exhaustifs d'espèces biologiques susceptibles d'être éliminées par le procédé selon l'invention, on peut citer tout type de micro-organismes tel que les bactéries, les spores notamment les spores de *Bacillus anthracis,* les virus, les champignons, les levures et les toxines. Les espèces ou espèces biologiques qui sont éliminé(e)s, détruit(e)s, inactivé(e)s, par la mousse selon l'invention sont essentiellement des espèces ou éléments bio-toxiques tel(le)s que les spores pathogènes comme, par exemple, les spores de *Bacillus anthracis,* les bactéries Gram - (comme par exemple *Yersinia pestis, Francisella tularensis, Pseudomonas aeruginosa, salmonella thyphimurium et Legionella sp*) et Gram + (*Staphylococcus aureus, Clostridium sp* et *Streptococcus sp*), les toxines comme, par exemple, la toxine botulique, la ricine ou la curcine, et les virus comme, par exemple, les virus des fièvres hémorragiques (de type Ebola par exemple) ou les coronavirus (de type SRAS par exemple).

Dans le procédé selon l'invention, la mise en contact entre la surface à traiter et la mousse aqueuse désinfectante est directe et peut être réalisée de différentes façons en mode « statique ».

Dans un 1$^{er}$ mode de réalisation, la mise en contact consiste à appliquer, sur la surface à traiter telle que précédemment définie, la mousse aqueuse désinfectante telle que précédemment définie. Ce 1$^{er}$ mode de réalisation peut impliquer une application par pulvérisation ou par talochage i.e. étalement par couche. Dans ce 1$^{er}$ mode de réalisation, la mousse peut être générée au moment de la mise en contact (pulvérisation) ou, au contraire, préalablement à cette mise en contact (talochage) et ce, dans des espaces ouverts ou fermés. La couche de mousse aqueuse désinfectante pulvérisée ou étalée peut être de 0,5 cm à 5 cm et avantageusement de 1 à 2 cm.

Dans un 2$^{nd}$ mode de réalisation, la mise en contact consiste à remplir des structures présentant une surface à traiter telle que précédemment définie avec la mousse aqueuse désinfectante telle que précédemment définie. Ces structures sont notamment des milieux clos ou semi-clos pouvant être de volume variable et important. Ce 2$^{nd}$ mode de réalisation est particulièrement adapté pour le traitement de lieux et d'infrastructures accessibles et inaccessibles à l'homme tels que des tuyaux d'aération ou des pièces contenant plusieurs éléments non déplaçables comme les sanitaires d'un avion. En effet, la mousse limite les volumes morts de liquide, en occupant tout l'espace et en mouillant toutes les surfaces comme les tuyaux, les grilles ou encore l'inter-espace entre des objets non amovibles.

La durée de la mise en contact s'étend de quelques minutes au séchage complet de la mousse aqueuse désinfectante. Avantageusement, la durée de la mise en contact est supérieure à 10 min, notamment supérieure à 20 min, et tout particulièrement supérieure à 30 min, qui est le temps adapté à la décontamination biologique et à l'utilisation.

Dans le procédé selon la présente invention, suite à l'étape de mise en contact, on laisse sécher ladite mousse pulvérisée ou talochée par évaporation ou drainer ladite mousse utilisée en remplissage.

De plus, le procédé selon la présente invention peut présenter, suite à l'étape de mise en contact, une étape visant à récupérer la mousse ou les résidus de cette mousse. Avantageusement, cette récupération peut se faire par aspiration. Dans le cas où il reste encore de la mousse i.e. avant séchage complet, c'est cette dernière qui est aspirée. Au contraire si on a laissé s'évaporer la part liquide de la mousse et donc, si on a laissé sécher la mousse, seuls des résidus secs non toxiques sont présents au niveau de la surface traitée et ce sont ces derniers qui sont récupérés par aspiration ou encore par essuyage notamment aux moyens d'une lingette ou d'une éponge. Comme ces résidus secs sont non toxiques, il est également possible de les laisser sur la surface traitée sans les récupérer.

D'autres caractéristiques et avantages de la présente invention apparaîtront encore à la lecture des exemples ci-après donnés à titre illustratif et non limitatif et faisant référence aux figures annexées.

## BRÈVE DESCRIPTION DES DESSINS

La Figure 1 présente l'évaluation moyennée de l'efficacité de décontamination d'une mousse selon l'invention contenant différentes concentrations d'hypochlorite de sodium (Figure 1A) ou différentes concentrations de peroxyde d'hydrogène (Figure 1B).

La Figure 2 présente la vitesse moyenne de décontamination d'une mousse selon l'invention contenant de l'hypochlorite de sodium (Figure 2A) ou du peroxyde d'hydrogène (Figure 2B).

La Figure 3 est une schématisation du protocole expérimental utilisé pour évaluer l'efficacité d'une mousse selon l'invention sur carreau de faïence.

La Figure 4 présente l'évaluation de l'efficacité de décontamination d'une mousse selon l'invention contenant de l'hypochlorite de sodium ou du peroxyde d'hydrogène sur carreau de faïence (Figure 4A) ou sur plaque d'aluminium (Figure 4B).

La Figure 5 présente l'évaluation de l'efficacité de décontamination d'une mousse selon l'invention contenant de l'hypochlorite de sodium ou du peroxyde d'hydrogène sur paroi verticale.

La Figure 6 présente l'évaluation de l'efficacité de décontamination d'une mousse selon l'invention contenant de l'hypochlorite de sodium ou du peroxyde d'hydrogène par remplissage.

La Figure 7 présente l'évaluation de l'efficacité de décontamination d'une mousse selon l'invention contenant de l'hypochlorite de sodium ou du peroxyde d'hydrogène et présentant différents pourcentages d'humidité.

La Figure 8 présente l'évaluation de l'efficacité de décontamination d'une mousse selon l'invention contenant de l'hypochlorite de sodium ou du peroxyde d'hydrogène après un vieillissement des solutions moussantes initiales.

La Figure 9 présente une comparaison de la moussabilité et du drainage à t=0, t=1 sem et t=5 sem de stockage, d'une mousse selon l'invention contenant de l'hypochlorite de sodium (Figure 9A) ou du peroxyde d'hydrogène (Figure 9B).

La Figure 10 présente la cinétique de glissement d'un dépôt d'une mousse selon l'invention contenant de l'hypochlorite de sodium ou du peroxyde d'hydrogène sur un tableau blanc d'écriture à feutres effaçables.

La Figure 11 présente les cinétiques d'évaporation d'une mousse neutre (Figure 11A) ou d'une mousse selon l'invention contenant de l'hypochlorite de sodium (Figure 11B) ou du peroxyde d'hydrogène (Figure 11C), l'ensemble de ces mousses pouvant contenir 1,5 g/l ou 3 g/l de xanthane, comme agent viscosant.

## EXPOSÉ DÉTAILLÉ DE MODES DE RÉALISATION PARTICULIERS

### I. Formulations utilisées pour la mousse.

Les différentes formulations des solutions moussantes utilisées lors des expérimentations présentées ci-après, sont reprises dans le Tableau 1 ci-après :

Tableau 1

| Nom de la formulation | Composition | Concentration par Litre de solution moussante |
|---|---|---|
| Neutre à 1,5 g/l de Xanthane | $H_2O$ | 839 ml/L |
| | Glucopon | 11 g/L |
| | Xanthane à 10 g/l | 150 ml/L |
| Neutre à 2 g/l de Xanthane | $H_2O$ | 789 ml/L |
| | Glucopon | 11 g/L |
| | Xanthane à 10 g/l | 200 ml/L |
| Neutre à 2,5 g/l de Xanthane | $H_2O$ | 739 ml/L |
| | Glucopon | 11 g/L |
| | Xanthane à 10 g/l | 250 ml/L |
| Neutre à 3 g/l de Xanthane | H2O | 689 ml/L |
| | Glucopon | 11 g/L |
| | Xanthane à 10 g/l | 300 ml/L |

(suite)

| Nom de la formulation | Composition | Concentration par Litre de solution moussante |
|---|---|---|
| NaOCl à 1% | H2O | 768 ml/L |
| | Glucopon | 11 g/L |
| | Xanthane à 10 g/l | 150 ml/L |
| | NaOCl à 14 % | 71 ml/L |
| NaOCl à 2% | H2O | 696 ml/L |
| | Glucopon | 11 g/L |
| | Xanthane à 10 g/l | 150 ml/L |
| | NaOCl à 14 % | 143 ml/L |
| NaOCl à 3% | H2O | 625 ml/L |
| | Glucopon | 11 g/L |
| | Xanthane à 10 g/l | 150 ml/L |
| | NaOCl à 14 % | 214 ml/L |
| NaOCl à 4% | H2O | 553 ml/L |
| | Glucopon | 11 g/L |
| | Xanthane à 10 g/l | 150 ml/L |
| | NaOCl à 14 % | 286 ml/L |
| NaOCl à 5% et 1,5 g/l de Xanthane | H2O | 482 ml/L |
| | Glucopon | 11 g/L |
| | Xanthane à 10 g/l | 150 ml/L |
| | NaOCl à 14 % | 357 ml/L |
| NaOCl à 5% et 2 g/l de Xanthane | H2O | 431 ml/L |
| | Glucopon | 11 g/L |
| | Xanthane à 10 g/l | 200 ml/L |
| | NaOCl à 14 % | 358 ml/L |
| NaOCl à 5% et 2,5 g/l de Xanthane | H2O | 381 ml/L |
| | Glucopon | 11 g/L |
| | Xanthane à 10 g/l | 250 ml/L |
| | NaOCl à 14 % | 358 ml/L |
| NaOCl à 5% et 3 g/l de Xanthane | H2O | 331 ml/L |
| | Glucopon | 11 g/L |
| | Xanthane à 10 g/l | 300 ml/L |
| | NaOCl à 14 % | 358 ml/L |
| NaOCl à 7,5% | H2O | 303 ml/L |
| | Glucopon | 11 g/L |
| | Xanthane à 10 g/l | 150 ml/L |
| | NaOCl à 14 % | 536 ml/L |

(suite)

| Nom de la formulation | Composition | Concentration par Litre de solution moussante |
|---|---|---|
| H2O2 à 1% | H2O | 806 ml/L |
| | Glucopon | 11 g/L |
| | Xanthane à 10 g/l | 150 ml/L |
| | H2O2 à 30 % | 33 ml/L |
| H2O2 à 2% | H2O | 772 ml/L |
| | Glucopon | 11 g/L |
| | Xanthane à 10 g/l | 150 ml/L |
| | H2O2 à 30 % | 67 ml/L |
| H2O2 à 5% et 1,5 g/l de Xanthane | H2O | 672 ml/L |
| | Glucopon | 11 g/L |
| | Xanthane à 10 g/l | 150 ml/L |
| | H2O2 à 30 % | 167 ml/L |
| H2O2 à 5% et 2 g/l de Xanthane | H2O | 622 ml/L |
| | Glucopon | 11 g/L |
| | Xanthane à 10 g/l | 200 ml/L |
| | H2O2 à 30 % | 167 ml/L |
| H2O2 à 5% et 2,5 g/l de Xanthane | H2O | 572 ml/L |
| | Glucopon | 11 g/L |
| | Xanthane à 10 g/l | 250 ml/L |
| | H2O2 à 30 % | 167 ml/L |
| H2O2 à 5% et 3 g/l de Xanthane | H2O | 523 ml/L |
| | Glucopon | 11 g/L |
| | Xanthane à 10 g/l | 300 ml/L |
| | H2O2 à 30 % | 167 ml/L |
| H2O2 à 8% | H2O | 574 ml/L |
| | Glucopon | 11 g/L |
| | Xanthane à 10 g/l | 150 ml/L |
| | H2O2 à 30 % | 265 ml/L |

## II. Protocole opératoire des essais biologiques.

Les essais sont réalisés avec des spores de *Bacillus thuringiensis* (Bt) qui est un simili de *Bacillus anthracis,* sous un PSM (Poste de Sécurité Microbiologique) consacré aux spores dans un laboratoire de microbiologie L2. Des boîtes de pétri sont contaminées par 100 μl d'une solution à $10^8$ spores de Bt/ml, soit un dépôt de $10^7$ spores de Bt, qui est laissée à sécher totalement sous la hotte (environ 1h30).

Les solutions moussantes sont préparées en laboratoire avec un générateur statique à billes. Les mousses sont générées dans un bécher de 2 litres puis pesées pour déterminer l'humidité de la mousse. Les mousses sont ensuite déposées sur les spores à l'aide d'une spatule.

Les mousses restent en contact avec les spores boîtes fermées, pendant 1h à 1h30 environ ou suivant le protocole d'essai (exemple pour la vitesse d'action biocide).

Pour chaque boîte, les spores sont reprises en déposant en plusieurs fois de l'eau stérile et placées dans un ou plusieurs tubes Falcon complétés jusqu'à 45 ml. Les tubes Falcon sont centrifugés 15 min à 4000 tr/min.

Le surnageant est retiré et le culot est re-suspendu dans 10 ml de milieu nutritif Luria-Broth (LB) liquide, puis passé au vortex. Si une même boîte a nécessité l'utilisation de plusieurs tubes Falcon, les tubes sont combinés en un. Les tubes Falcon sont placés en étuve à 30°C pendant 1h.

Ce passage à l'étuve dans le milieu LB permet l'initiation de la désporulation des spores de *Bacillus thuringiensis* qui se transforment en forme végétative à bonne température, et la mise en contact prolongée du support avec le milieu afin de récupérer un maximum de spores présentes sur le support. Ces formes végétatives peuvent pousser sous forme de colonies sur un milieu nutritif solide (gélose) en boîte de pétri et ainsi être visuellement comptées. Cela permet une estimation du nombre de spores initiales inactivées.

Pour chacun des tubes incubés à 30°C, une gamme de dilutions successives en volume au dixième (de dixième en dixième) est réalisée avec du LB liquide (dilution jusqu'à $10^8$ ou au cent millionnième). Enfin, 1 ml est prélevé dans chacun des tubes de chaque gamme de dilutions, puis est déposé au fond d'une boîte de pétri vide et stérile.

Du milieu LB gélosé est ensuite coulé dans la boîte (ensemencement dans la masse). Les boîtes sont ensuite placées dans un incubateur à 30°C pendant environ 20h. Les colonies sont comptées une à une et une moyenne de spores vivantes est calculée. Un essai ne contenant pas de désinfectant, appelé Neutre, est réalisé au minimum une fois pour chaque essai, de façon à contrôler le bon déroulement du protocole opératoire.

### III. Evaluation de l'efficacité biocide de la formulation.

III.1. Mousses à différentes concentrations en hypochlorite de sodium et en peroxyde d'hydrogène.

Des essais sont réalisés afin de déterminer l'efficacité biocide à différentes concentrations en NaOCl et en $H_2O_2$. Ces essais sont réalisés suivant le protocole opératoire précédemment décrit et suivant les formulations précédemment détaillées.

Ainsi, trois essais ont été réalisés pour NaOCl à 5%, deux essais pour $H_2O_2$ à 5% et un essai pour NaOCl à 1%, 2%, 3%, 4% et 7,5%, ainsi que pour $H_2O_2$ à 1% et 2%.

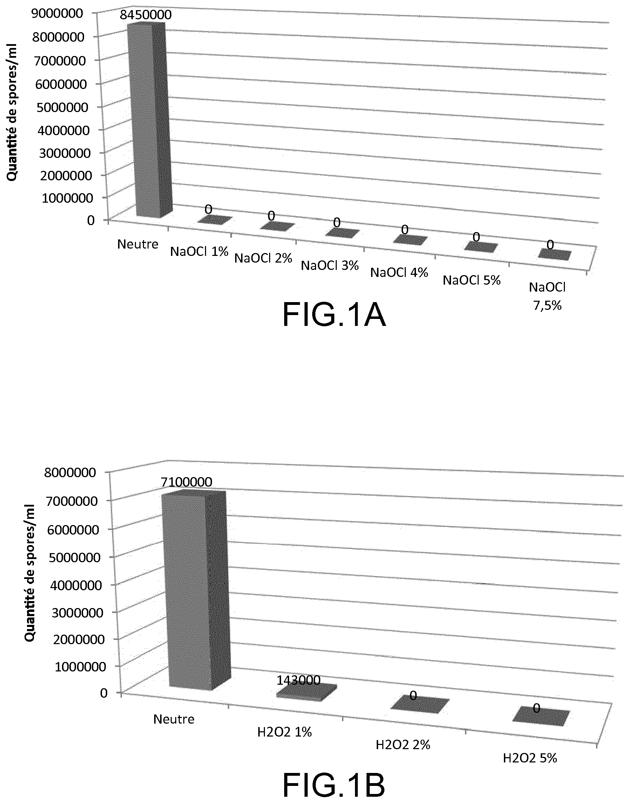

Les résultats de ces essais sont présentés dans les Figures 1A et 1B. Il en ressort que la mousse selon la présente invention avec l'hypochlorite de sodium est efficace à partir d'une concentration de 1% en hypochlorite de sodium et que celle au peroxyde d'hydrogène est efficace à partir d'une concentration de 2% en peroxyde d'hydrogène.

III.2. Evaluation de la vitesse de décontamination des mousses.

Des essais sont réalisés afin de déterminer la vitesse d'efficacité biocide des mousses NaOCl et $H_2O_2$. Ces essais sont réalisés suivant le protocole opératoire précédemment décrit.

Le temps de contact entre la mousse et les spores est mesuré avec un chronomètre. Ont été testés des temps de contact mousse/contamination de 30 s, 5 min, 7 min, 10 min, 15 min, 30 min, 45 min et 1 h. Il faut cependant prendre en compte le temps de traitement incompressible dû au protocole expérimental (récupération de la mousse et centrifugation) d'environ 20 min. La mousse récupérée est diluée avec de l'eau stérilisée donc le désinfectant est en plus faible concentration et la mousse est désagrégée.

Le nombre de reproductions de ces essais est présenté dans le Tableau 2 ci-après :

Tableau 2

| Temps de contact | Nombre d'essai NaOCl 5% | Nombre d'essai $H_2O_2$ 5% |
|---|---|---|
| 30 secondes | 2 | 2 |
| 5 minutes | 1 | - |
| 7 minutes | 2 | 2 |
| 10 minutes | 1 | - |
| 13 minutes | 2 | 1 |
| 15 minutes | 2 | 3 |
| 30 minutes | 2 | 2 |
| 45 minutes | 2 | 2 |
| 60 minutes | 4 | 4 |

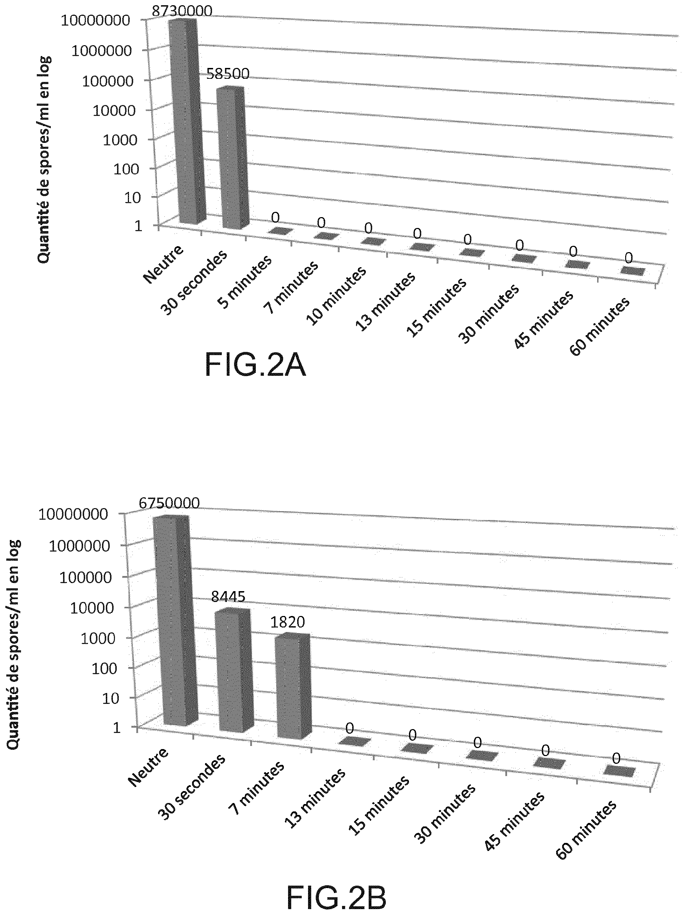

Les résultats de ces essais sont présentés aux Figures 2A et 2B. Ainsi, la mousse à l'hypochlorite de sodium 5% et celle au peroxyde d'hydrogène 5% neutralisent respectivement l'ensemble des spores (environ $10^7$ spores) à partir de 5 min et de 13 min de contact. Il faut ajouter, à ces temps de contact, le temps de traitement dû au protocole expérimental qui permet d'affirmer que les solutions sont efficaces en 30 min donc, globalement, les deux désinfectants sont efficaces en moins d'une heure.

III.3. Evaluation de l'efficacité de décontamination des mousses sur différents supports.

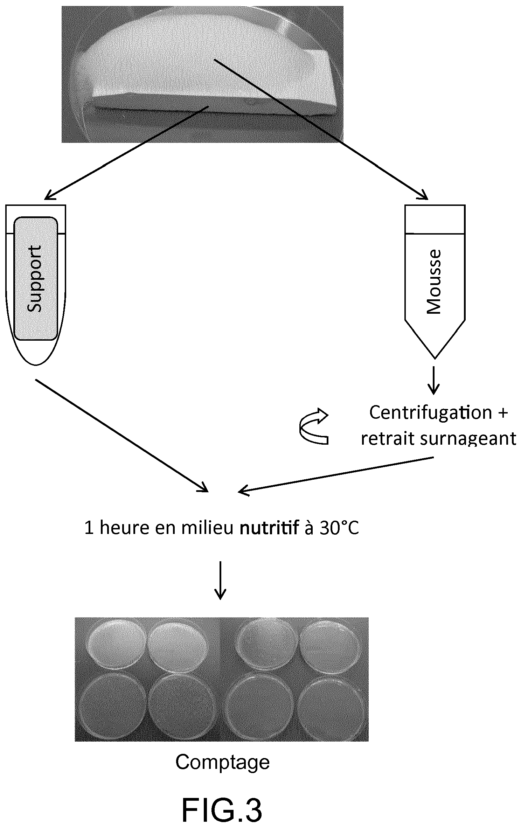

Des essais sont réalisés afin de déterminer l'efficacité biocide des mousses NaOCl et $H_2O_2$ sur différents matériaux. Les essais suivent le protocole opératoire précédemment décrit hormis les dépôts qui se font sur un carreau de faïence ou sur une plaque d'aluminium posé(e) dans une boîte de pétri avec un temps de contact entre la mousse et le matériel contaminé de 30 min (Figure 3).

Le matériel contaminé est placé dans un tube avec 30 ml de milieu nutritif Luria-Broth (LB) liquide et mis en étuve 1 h à 30°C. Ce passage à l'étuve permet d'initier la désporulation des spores de *Bacillus thuringiensis* qui se transforment en forme végétative et de prolonger le temps de contact avec le support afin de vérifier qu'il ne reste pas de spores sur le carreau de faïence ou d'aluminium.

Pour chacun des tubes incubés à 30°C, une gamme de dilutions successives en volume au dixième (de dixième en dixième) est réalisée avec du LB liquide (dilution jusqu'à $10^8$ ou au cent millionième). Enfin, 1 ml est prélevé dans chacun des tubes de chaque gamme de dilutions, puis est déposé au fond d'une boîte de pétri vide et stérile. Du milieu LB gélosé est ensuite coulé dans la boîte (ensemencement dans la masse). Les boîtes sont ensuite placées dans un incubateur à 30°C pendant environ 20 h. Les colonies sont comptées une à une et une moyenne de spores vivantes est calculée. Le facteur de décontamination peut être calculé en déterminant l'abattement en milliers de spores tuées ($\log_{10}$).

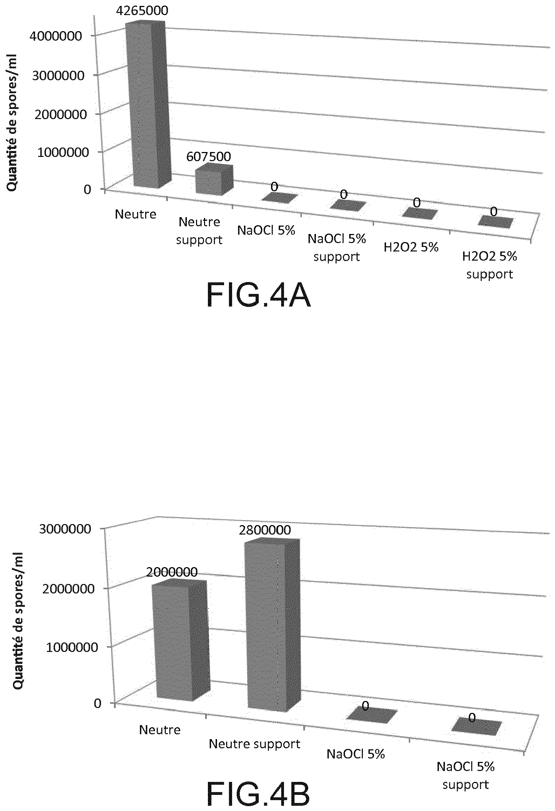

Les résultats de ces essais réalisés sur carreau de faïence ou plaque d'aluminium sont présentés aux Figures 4A et 4B. Les mousses selon l'invention à l'hypochlorite de sodium 5% et au peroxyde d'hydrogène 5% sont efficaces sur les carreaux de faïence en 30 min. Sur les plaques d'aluminium, seule la javel en tant qu'agent désinfectant a été testée et une mousse selon l'invention contenant de l'hypochlorite de sodium 5% est également efficace en 30 min sur un tel support.

III.4. Evaluation de l'efficacité de décontamination des mousses sur paroi verticale.

Des essais sont réalisés afin de déterminer l'efficacité biocide des mousses NaOCl et $H_2O_2$ sur une paroi verticale. Ces essais sont réalisés suivant le protocole opératoire précédemment exposé avec des boîtes rectangulaires et suivant les formulations précédemment détaillées.

Pour ces essais sur paroi verticale, la mousse déposée forme un cône dont la base repose sur le fond de la boîte et remonte sur la paroi verticale. La zone de contamination reste couverte par la mousse.

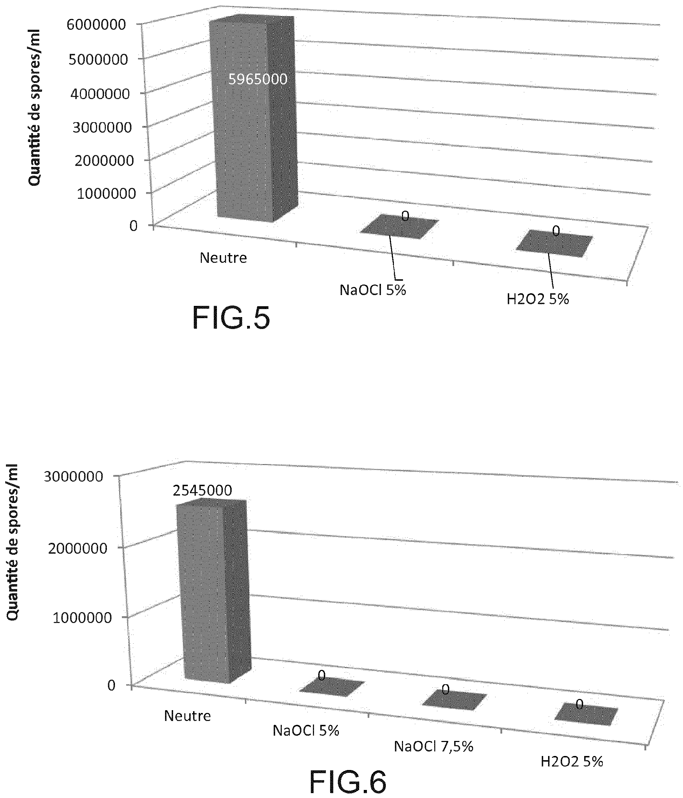

Pour les essais sur paroi verticale, un essai avec une mousse NaOCl à 5% et deux essais avec une mousse $H_2O_2$ à 5% ont été réalisés. Les résultats de ces essais sont présentés Figure 5. Les mousses NaOCl à 5% et $H_2O_2$ à 5% ont des propriétés physico-chimiques ainsi qu'une vitesse d'action leur permettant d'avoir un temps de contact suffisant avec la paroi verticale pour décontaminer cette dernière.

III.5. Evaluation de l'efficacité de décontamination des mousses en remplissage.

Des essais sont réalisés afin de déterminer l'efficacité biocide des mousses NaOCl et $H_2O_2$ en remplissage. Ces essais sont réalisés suivant le protocole opératoire précédemment décrit avec des contenants pouvant être des boîtes rectangulaires ou des tubes et suivant les formulations précédemment détaillées. La contamination se fait sur deux des parois verticales de la boîte (50 $\mu$l de solution à $10^8$ spores/ml sur chaque paroi) ou sur la paroi verticale du tube.

Pour les essais en remplissage, six essais avec une mousse NaOCl à 5%, un essai avec une mousse NaOCl à 7,5% et cinq essais avec une mousse $H_2O_2$ à 5% ont été réalisés. Les résultats de ces essais sont présentés Figure 6. Les différents types de mousse testés permettent de décontaminer des enceintes closes par remplissage.

III.6. Evaluation de l'efficacité de la décontamination des mousses en fonction du pourcentage d'humidité.

Des essais sont réalisés afin de déterminer l'efficacité biocide à différents pourcentages d'humidité des mousses NaOCl et $H_2O_2$. Ces essais sont réalisés suivant le protocole opératoire biologique et suivant les formulations précédemment détaillés.

Le pourcentage d'humidité de la mousse est modifié suite aux changements de paramètres du générateur de mousse. Le générateur est ainsi paramétré une première fois pour obtenir des mousses à 2,5% d'humidité et une seconde fois pour obtenir 3% d'humidité. Un essai a été réalisé avec une mousse NaOCl à 3,5% d'humidité et une autre à 4%. Il a en été de même pour une mousse $H_2O_2$ où un essai a été fait à 2,7% et un autre à 2,8% d'humidité.

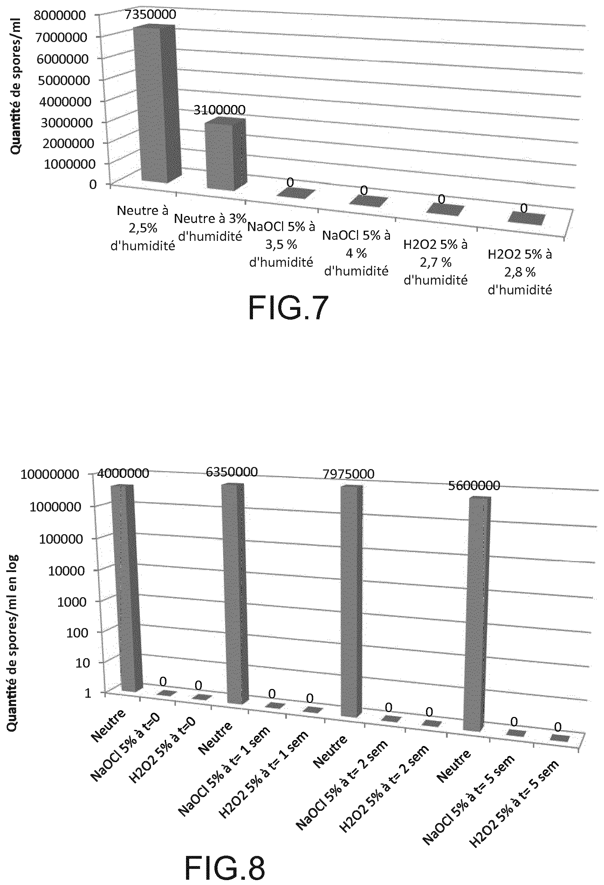

Les résultats de ces essais sont présentés Figure 7. La mousse NaOCl est efficace dès 3,5% d'humidité (foisonnement 28,5) et la mousse $H_2O_2$ dès 2,7% d'humidité (foisonnement 37).

11.7. Evaluation de l'efficacité de la décontamination des mousses après un vieillissement des solutions.

Des essais sont réalisés afin de déterminer l'efficacité biocide des mousses NaOCl et $H_2O_2$ après plusieurs semaines de stockage. Ces essais sont réalisés suivant le protocole opératoire biologique et les formulations précédemment détaillés.

Des essais sont réalisés le jour de la préparation des solutions (t=0), 1 semaine après (t=1 sem), 2 semaines après (t=2 sem) et 5 semaines après leur préparation (t=5 sem). Les solutions liquides initiales à partir desquelles sont réalisées les mousses, sont stockées en chambre froide à 4°C le temps du vieillissement.

Un essai est réalisé avec une mousse NaOCl à 5% et une mousse $H_2O_2$ à 5% à chaque temps de vieillissement. Les résultats de ces essais sont présentés Figure 8. Les mousses gardent la capacité de décontamination des spores de Bt même après 5 semaines de stockage de la solution moussante initiale.

III.8. Evaluation de la moussabilité des solutions moussantes et stabilité dans le temps des mousses correspondantes.

Des expériences de moussage sont réalisées afin de déterminer la moussabilité des solutions de NaOCl et de $H_2O_2$ après plusieurs semaines de stockage. Ces expériences sont réalisées à l'aide d'un appareil commercial dénommé Foamscan de la société Teclis. Il permet de mesurer, dans une colonne de 150 ml et avec un volume de solution initial de 20 ml, non seulement la vitesse de moussage pour un débit d'air donné (déterminé à 100 ml) mais aussi la stabilité de la mousse en arrêtant l'injection d'air.

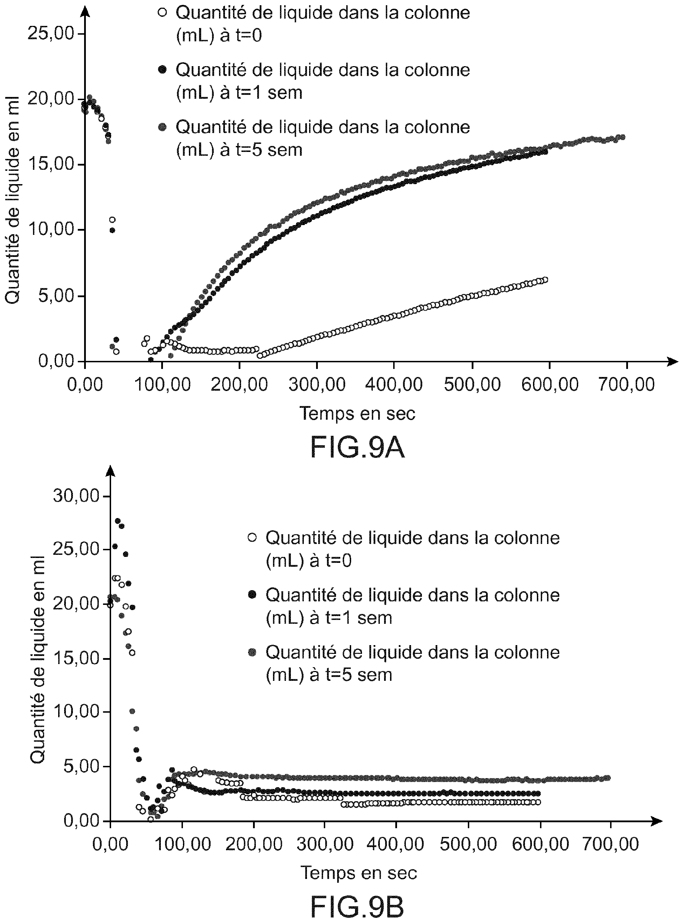

On mesure donc, en fonction du temps, la diminution de la quantité de liquide dans la colonne jusqu'à l'arrêt de l'injection d'air (environ 60 secondes, cf Figures 9A et 9B). Puis, à partir de l'arrêt du gaz, la quantité de liquide dans la colonne augmente plus ou moins vite, traduisant le drainage de la mousse donc sa stabilité.

En présence d'un agent viscosant comme le xanthane, on peut observer un retard au démarrage du drainage qui se traduit par l'existence d'un plateau avant l'augmentation du niveau de liquide dans la colonne. La durée de ce plateau traduit la stabilité de la mousse. Les résultats de vieillissement obtenus à t=0, t= 1 sem et t= 5 sem, sont illustrés Figure 9.

Ainsi, pour la solution d'hypochlorite de sodium à 5% et une quantité de xanthane de 1,5 g/l (Figure 9A), l'ensemble des courbes montre un transfert (première partie de la courbe) identique et total du liquide dans la mousse. La diminution est linéaire traduisant que l'intégralité de l'air injecté est piégé pour former la mousse. Après coupure de l'air, la mousse fraîche (t=0 sem) présente un plateau d'environ 180 s, puis le drainage commence lentement : après 10 min, seulement 25% du liquide a drainé. Après 1 semaine de stockage, si la moussabilité est identique, la stabilité, par contre, est plus faible : le plateau du retard au drainage observé n'est plus que de 40 s et le drainage est plus rapide (75% du liquide draine en 10 min). Les tests d'efficacité de décontamination ont montré que la solution biocide reste moussante et que la mousse NaOCl correspondante reste active après 5 semaines de stockage.

Pour la solution de peroxyde d'hydrogène à 5% et une quantité de xanthane de 1,5 g/l (Figure 9B), l'ensemble des courbes montre un transfert (première partie de la courbe) identique et total du liquide dans la mousse. La diminution est linéaire traduisant que l'intégralité de l'air injecté est piégé pour former la mousse. Après coupure de l'air, l'ensemble des mousses présente un retard au drainage supérieur à 10 min. Les mousses $H_2O_2$ sont plus stables que les mousses NaOCl.

111.9. Evaluation de la tenue des mousses sur une paroi verticale suivant différentes concentrations en xanthane.

Ces essais visent à déterminer la tenue de dépôts de mousse sur une paroi verticale en mesurant le glissement de la mousse par rapport au temps. Des dépôts de mousse de 3 tailles différentes ont été réalisés avec les différentes solutions de la présente invention et avec différentes concentrations en xanthane qui est l'agent viscosant de la formulation. Pour ces essais, quatre concentrations de xanthane sont testées : 1,5 g/l, 2 g/l, 2,5 g/l et 3 g/l.

Ces essais sont réalisés avec, comme support, un tableau blanc d'écriture à feutres effaçables. C'est une matière non poreuse et très lisse qui permet de réaliser ces essais dans les conditions les plus difficiles. La position du dépôt à t = 0 est marquée et, au bout de différents temps, la distance de glissement de la mousse est mesurée.

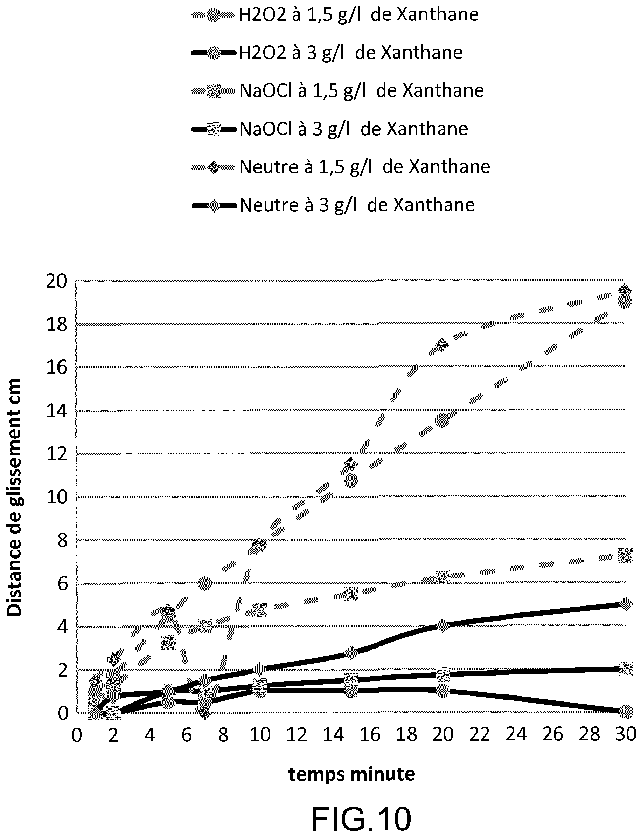

Les mousses NaOCl et $H_2O_2$ adhèrent à la paroi d'un tableau blanc d'écriture à feutres effaçables. Le glissement de la mousse est fortement ralenti (moyenne de glissement de 3 cm en 30 min) quand la concentration en xanthane est augmentée à 3 g/l (Figure 10).

III.10. Evaluation de l'évaporation des différentes mousses.

Des essais sont réalisés afin de déterminer la vitesse d'évaporation d'une couche de mousse et l'influence des agents désinfectant sur celle-ci. Ces essais sont réalisés à l'aide d'une enceinte climatique à température et hygrométrie maîtrisées. Il s'agit d'évaporer horizontalement environ 24 cm$^3$ (4 cm x 4 cm x 1,5 cm) de mousse dans une nacelle en inox en mesurant la perte de masse au cours du temps, à l'aide d'une balance de précision. L'enceinte climatique est réglée à une température de 22°C et une humidité relative de 40%.

La mousse est générée par un générateur statique à billes et déposée dans la nacelle en inox. Celle-ci est ensuite placée dans la balance se trouvant dans l'enceinte climatique. La balance est fermée entièrement. Elle indique la masse de la mousse contenue dans la nacelle, le tarage de la nacelle ayant été préalablement effectué. Un logiciel permet d'enregistrer, toutes les 10 min, la masse mesurée par la balance en temps réel. Un second logiciel permet de prendre des photos de l'intérieur de la balance.

Les mousses générées pour ces essais sont des mousses NaOCl à 5%, $H_2O_2$ à 5% et neutre à 1,5 g/l ou 3 g/l de xanthane. La durée des essais est d'une nuit ou d'un week-end.

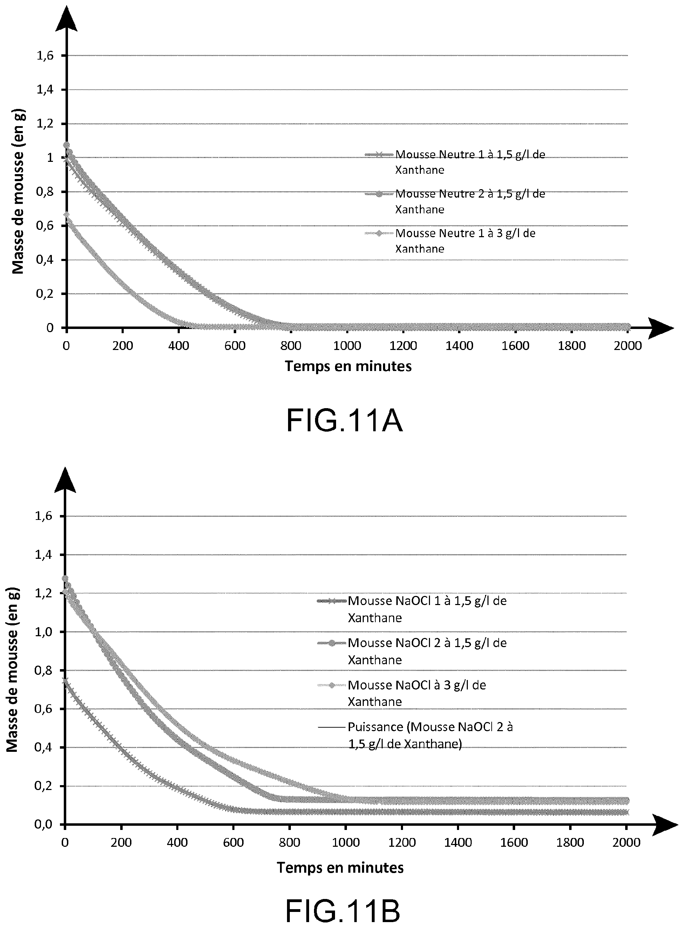

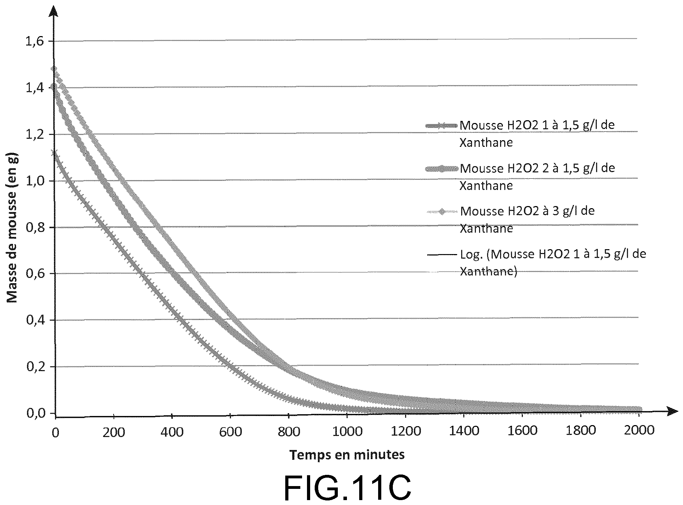

Les résultats de ces essais sont présentés Figure 11 avec les essais pour les mousses neutres, les mousses NaOCl et les mousses $H_2O_2$ respectivement aux Figures 11A, 11B et 11C. La vitesse d'évaporation est constante sur au moins 300 min puis commence à baisser pour une masse de mousse faible. On constate aussi qu'avec les mousses NaOCl (Figure 11B), il reste, après évaporation, un résidu de cristaux de chlorure de sodium et de carbonate de sodium dont la masse est faible mais mesurable. En calculant l'équation de la pente sur les 300 premières minutes (régression linéaire), on constate que les coefficients directeurs des pentes de l'ensemble des essais d'évaporation sont relativement proches (moyenne -0,0019 +/- 0,0003) que ce soit avec ou sans désinfectant et aux deux concentrations de xanthane. Un essai avec une couche d'eau a été réalisé et l'évaporation présente la même cinétique. L'évaporation au début ne semble donc pas influencée par la formulation de la mousse et correspond à l'évaporation de l'eau. Pour 1 g de mousse étalée sur 4 cm par 4 cm et sur une épaisseur d'1,5 cm, l'évaporation se fait en moyenne en 9 h.

Ces essais ont été complétés par le dépôt d'une couche de mousse NaOCl à 5% déposée à la spatule sur une surface verticale en plastique contaminée par des tâches de spores Bt. Cette expérience a été conduite sous sorbonne ventilée dont le flux d'air accélère la vitesse d'évaporation.

Les mesures d'efficacité de décontamination sur spores de Bt, suivant le protocole opératoire biologique précédent, montrent une excellente efficacité (supérieur à 10$^6$ spores inactivées).

Ainsi, en 14 h, la mousse en couche a entièrement disparu et le liquide de la mousse s'est évaporé. Après évaporation, on retrouve, pour chaque mousse, un fin film transparent de glucopon et de xanthane. De plus, pour la mousse NaOCl, on trouve également de petits cristaux de chlorure de sodium et de carbonate de sodium résultants de la réaction d'évaporation.

III.11. Evaluation de la récupération de la mousse par aspiration.

Des essais de récupération de la mousse par aspiration ont été réalisés avec un aspirateur pour liquide.

A cet effet, une couche de 1 à 3 cm d'épaisseur d'une mousse selon l'invention a été appliquée par talochage sur une paroi verticale ou un bac de 30 l a été rempli par une mousse selon l'invention. Dans ces deux modes d'applications i.e. talochage ou remplissage, la mousse est aspirable.

**RÉFÉRENCES**

**[1]** EFT Holdings Inc. "MATERIAL SAFETY DATA SHEET NAME OF FINISHED SOLUTION: EasyDECON® DF200-531X" Alabama 2008. http://www.easydecon.com/easydecon/EasyDECON%20DF200%20MSDS.pdf
**[2]** Demande de brevet US 7,276,468 au nom de Sandia Corporation, délivré le 2 octobre 2007.
**[3]** EFT Holdings Inc. "Performance Data" Alabama 2011. http://www.easydecon.com/easydecon/FactSheete248.html
**[4]** Allen Vanguard - CASCAD™ Decontamination Foam, 2009. http://reports.hms-online.org/ViewProduct.aspx?CategoryId=175&ProductId=721
**[5]** "Biological Agent Decontamination Technology Testing" U.S. EPA. Biological Agent Decontamination Technology Testing . U.S. Environmental Protection Agency, Washington, DC, EPA/600/R-10/087, 2010.
**[6]** Demande internationale WO 2004/008463 au nom du CEA et de la COGEMA, publiée le 22 janvier 2004.
**[7]** "A quantitative kinetic theory of emulsion type, I. Physical chemistry of the emulsifying agent" Gas/Liquid and Liquid/Liquid Interface. Proceedings of the International Congress of Surface Activity (1957): 426-438.
**[8]** Règlement (UE) n°528/2012 du 22 mai 2012 concernant la mise à disposition sur le marché et l'utilisation des produits biocides.

**[9]** Demande internationale WO 02/043847 au nom du CEA, publiée le 6 juin 2002.

**Revendications**

1. Mousse consistant en une dispersion de bulles de gaz dans une solution moussante constituée :

   - de 0,05 à 1,5% en masse d'un agent tensioactif organique moussant ou d'un mélange d'agents tensioactifs organiques moussants,
   - de 0,05% à 0,8% en masse d'un agent gélifiant ou viscosant, organique ou d'un mélange d'agents gélifiants ou viscosants, organiques,
   - de 1% à 14% en volume d'un agent désinfectant ou d'un mélange d'agents désinfectants et
   - de l'eau,

   ladite mousse présentant un foisonnement compris entre 20 et 50.

2. Mousse selon la revendication 1, **caractérisée en ce que** ledit agent tensioactif organique moussant ou lesdits agents tensioactifs organiques moussants est/sont choisi(s) parmi les tensioactifs moussants non ioniques, les tensioactifs moussants anioniques et les tensioactifs moussants cationiques.

3. Mousse selon la revendication 1 ou 2, **caractérisée en ce que** ledit agent gélifiant ou viscosant, organique ou lesdits agents gélifiants ou viscosants, organiques est/sont choisi(s) parmi les polymères hydrosolubles, les hydrocolloïdes, les hétéropolysaccharides, les dérivés cellulosiques et les polysaccharides.

4. Mousse selon l'une quelconque des revendications 1 à 3, **caractérisée en ce que** ledit agent désinfectant ou lesdits agents désinfectants est/sont choisi(s) parmi les produits chlorés, les aldéhydes et les oxydants.

5. Mousse selon l'une quelconque des revendications 1 à 4, **caractérisée en ce que** ladite solution moussante est constituée :

   - de 0,1 à 1,1% en masse d'un agent tensioactif organique moussant ou d'un mélange d'agents tensioactifs organiques moussants,
   - de 0,15% à 0,3% en masse d'un agent gélifiant ou viscosant, organique ou d'un mélange d'agents gélifiants ou viscosants, organiques,
   - de l'eau et
   - soit de 2% à 7,5% en volume et notamment de l'ordre de 5% en volume d'un agent désinfectant ou d'un mélange d'agents désinfectants
   - soit de 5% à 14% en volume et notamment de l'ordre de 12% en volume d'un agent désinfectant ou d'un mélange d'agents désinfectants.

6. Mousse selon l'une quelconque des revendications 1 à 4, **caractérisée en ce que** ladite solution moussante est constituée :

   - de 0,1 à 1,1% en masse d'un alkylpolyglucoside,
   - de 0,15% à 0,3% en masse de gomme de xanthane,
   - de l'eau et
   - soit de 2% à 7,5% en volume et notamment de l'ordre de 5% en volume d'hypochlorite de sodium ou de peroxyde d'hydrogène,
   - soit de 5% à 14% en volume et notamment de l'ordre de 12% en volume d'hypochlorite de sodium ou de peroxyde d'hydrogène.

7. Procédé pour traiter une surface susceptible d'être contaminée par au moins un agent biologique consistant à mettre en contact ladite surface avec une mousse telle que définie à l'une quelconque des revendications 1 à 6.

8. Procédé selon la revendication 7, **caractérisé en ce que** ladite surface est en métal, en alliage métallique, en acier, en fer-blanc, en silicium, en verre contenant généralement des silicates, en verre de silice, en céramique, en brique, en porcelaine, en ciment, en béton, en asphalte, en pierre, en granit, en bois, en terre, en plastique ou en une quelconque de leurs associations.

9.  Procédé selon la revendication 7 ou 8, **caractérisé en ce que** ledit agent biologique est une espèce ou un élément bio-toxique choisi(e) dans le groupe constitué par les spores pathogènes, les bactéries Gram -, les bactéries Gram +, les toxines et les virus.

10. Procédé selon l'une quelconque des revendications 7 à 9, **caractérisé en ce que** la mise en contact consiste à :

    - appliquer, sur ladite surface, ladite mousse par pulvérisation ou par talochage ou
    - remplir des structures présentant ladite surface avec ladite mousse.

11. Procédé selon l'une quelconque des revendications 7 à 10, **caractérisé en ce que**, suite à ladite mise en contact, on laisse :

    - sécher ladite mousse pulvérisée ou talochée par évaporation
    - drainer ladite mousse utilisée en remplissage.

12. Procédé selon l'une quelconque des revendications 7 à 11, **caractérisé en ce que**, suite à ladite mise en contact, on récupère soit la mousse par aspiration avant séchage complet soit les résidus secs de cette mousse par aspiration ou par essuyage.

**Patentansprüche**

1.  Schaum, bestehend aus einer Dispersion von Gasblasen in einer schaumbildenden Lösung, bestehend aus:

    - 0,05 bis 1,5 Masse-% eines schaumbildenden, organischen Tensids oder eines Gemisches aus schaumbildenden, organischen Tensiden,
    - 0,05 bis 0,8 Masse-% eines organischen Geliermittels oder viskosmachenden Mittels oder eines Gemisches aus organischen Geliermitteln oder viskosmachenden Mitteln,
    - 1 Vol.-% bis 14 Vol.-% eines Desinfektionsmittels oder eines Gemisches aus Desinfektionsmitteln und
    - Wasser,
    wobei der Schaum einen Aufschlag zwischen 20 und 50 aufweist.

2.  Schaum nach Anspruch 1, **dadurch gekennzeichnet, dass** das schaumbildende organische Tensid bzw. die schaumbildenden organischen Tenside ausgewählt ist/sind aus nicht ionischen schaumbildenden Tensiden, anionischen schaumbildenden Tensiden und kationischen schaumbildenden Tensiden.

3.  Schaum nach Anspruch 1 oder 2, **dadurch gekennzeichnet, dass** das organische Geliermittel oder viskosmachende Mittel bzw. die organischen Geliermittel oder viskosmachenden Mittel ausgewählt ist/sind aus wasserlöslichen Polymeren, Hydrokolloiden, Heteropolysacchariden, Cellulosederivaten und Polysacchariden.

4.  Schaum nach einem der Ansprüche 1 bis 3, **dadurch gekennzeichnet, dass** das Desinfektionsmittel bzw. die Desinfektionsmittel ausgewählt ist/sind aus chlorierten Produkten, Aldehyden und Oxidationsmitteln.

5.  Schaum nach einem der Ansprüche 1 bis 4, **dadurch gekennzeichnet, dass** die schaumbildende Lösung besteht aus:

    - 0,1 bis 1,1 Masse-% eines schaumbildenden, organischen Tensids oder eines Gemisches aus schaumbildenden, organischen Tensiden,
    - 0,15 Masse-% bis 0,3 Masse-% eines organischen Geliermittels oder viskosmachenden Mittels oder eines Gemisches aus organischen Geliermitteln oder viskosmachenden Mitteln,
    - Wasser und
    - entweder 2 Vol.-% bis 7,5 Vol.-% und insbesondere etwa 5 Vol.-% eines Desinfektionsmittels oder eines Gemisches aus Desinfektionsmitteln
    - oder 5 Vol.-% bis 14 Vol.-% und insbesondere etwa 12 Vol.-% eines Desinfektionsmittels oder eines Gemisches aus Desinfektionsmitteln.

6.  Schaum nach einem der Ansprüche 1 bis 4, **dadurch gekennzeichnet, dass** die schaumbildende Lösung besteht aus:

- 0,1 bis 1,1 Masse-% eines Alkylpolyglucosids,
- 0,15 Masse-% bis 0,3 Masse-% Xanthangummi,
- Wasser und
- entweder 2 Vol.-% bis 7,5 Vol.-% und insbesondere etwa 5 Vol.-% Natriumhypochlorit oder Wasserstoffperoxid,
  - oder 5 Vol.-% bis 14 Vol.-% und insbesondere etwa 12 Vol.-% Natriumhypochlorit oder Wasserstoffperoxid.

**7.** Verfahren zum Behandeln einer Oberfläche, die mit zumindest einem biologischen Mittel kontaminiert sein kann, das darin besteht, die Oberfläche mit einem Schaum nach einem der Ansprüche 1 bis 6 in Kontakt zu bringen.

**8.** Verfahren nach Anspruch 7, **dadurch gekennzeichnet, dass** die Oberfläche aus Metall, Metalllegierung, Stahl, Weißblech, Silicium, weitgehend silikathaltigem Glas, Quarzglas, Keramik, Klinker, Porzellan, Zement, Beton, Asphalt, Stein, Granit, Holz, Erde, Kunststoff oder aus einer ihrer Verbindungen besteht.

**9.** Verfahren nach Anspruch 7 oder 8, **dadurch gekennzeichnet, dass** das biologische Mittel ein(e) biotoxische(s) Spezies oder Element, ausgewählt aus der Gruppe umfassend pathogene Sporen, gramnegative Bakterien, grampositive Bakterien, Toxine und Viren, ist.

**10.** Verfahren nach einem der Ansprüche 7 bis 9, **dadurch gekennzeichnet, dass** das Inkontaktbringen darin besteht,

- den Schaum durch Zerstäuben oder Abziehen auf die Oberfläche aufzutragen, oder
- die Oberfläche aufweisende Strukturen mit dem Schaum zu füllen.

**11.** Verfahren nach einem der Ansprüche 7 bis 10, **dadurch gekennzeichnet, dass** nach dem Inkontaktbringen

- der zerstäubte oder abgezogene Schaum durch Verdampfung getrocknet wird,
- der zum Füllen verwendete Schaum entwässert wird.

**12.** Verfahren nach einem der Ansprüche 7 bis 11, **dadurch gekennzeichnet, dass** nach dem Inkontaktbringen entweder der Schaum durch Absaugen vor dem vollständigen Trocken gewonnen wird oder die trockenen Rückstände dieses Schaums durch Absaugen oder Abwischen gewonnen werden.

**Claims**

**1.** Foam consisting of a dispersion of gas bubbles in a foaming solution consisting of:

- from 0.05% to 1.5% by weight of a foaming organic surfactant or of a mixture of foaming organic surfactants,
- from 0.05% to 0.8% by weight of an organic gelling or viscosifying agent or of a mixture of organic gelling or viscosifying agents,
- from 1% to 14% by volume of a disinfecting agent or of a mixture of disinfecting agents and
- water,
  said foam having an expansion between 20 and 50.

**2.** Foam according to claim 1, **characterised in that** said foaming organic surfactant or said foaming organic surfactants is/are chosen from non-ionic foaming surfactants, anionic foaming surfactants and cationic foaming surfactants.

**3.** Foam according to claim 1 or 2, **characterised in that** said organic gelling or viscosifying agent or said organic gelling or viscosifying agents is/are chosen from watersoluble polymers, hydrocolloids, heteropolysaccharides, cellulose derivatives and polysaccharides.

**4.** Foam according to any one of claims 1 to 3, **characterised in that** said disinfecting agent or said disinfecting agents is/are chosen from chlorinated products, aldehydes and oxidants.

**5.** Foam according to any one of claims 1 to 4, **characterised in that** said foaming solution consists of:

- from 0.1 to 1.1% by weight of a foaming organic surfactant or of a mixture of foaming organic surfactants,
- from 0.15% to 0.3% by weight of an organic gelling or viscosifying agent or of a mixture of organic gelling or viscosifying agents,

- water and
- either from 2% to 7.5% by volume and particularly of the order of 5% by volume of a disinfecting agent or of a mixture of disinfecting agents
- or from 5% to 14% by volume and particularly of the order of 12% by volume of a disinfecting agent or of a mixture of disinfecting agents.

6. Foam according to any one of claims 1 to 4, **characterised in that** said foaming solution consists of:

- from 0.1 to 1.1% by weight of an alkylpolyglucoside,
- from 0.15% to 0.3% by weight of xanthan gum,
- water and
- either from 2% to 7.5% by volume and particularly of the order of 5% by volume of sodium hypochlorite or hydrogen peroxide,
- or from 5% to 14% by volume and particularly of the order of 12% by volume of sodium hypochlorite or hydrogen peroxide.

7. Method for treating a surface likely to be contaminated with at least one biological agent consisting of contacting said surface with a foam as defined in any one of claims 1 to 6.

8. Method according to claim 7, **characterised in that** said surface is made of metal, metal alloy, steel, tinplate, silicon, glass generally containing silicates, silica glass, ceramic, brick, porcelain, cement, concrete, asphalt, stone, granite, wood, clay, plastic or any one of the combinations thereof.

9. Method according to claim 7 or 8, **characterised in that** said biological agent is a species or a bio-toxic element chosen in the group consisting of pathogenic spores, Gram - bacteria, Gram + bacteria, toxins and viruses.

10. Method according to any one of claims 7 to 9, **characterised in that** the contacting consists of:

- applying, on said surface, said foam by spraying or by floating or
- filling structures containing said surface with said foam.

11. Method according to any one of claims 7 to 10, **characterised in that**, following said contacting:

- said sprayed or floated foam is allowed to dry by evaporation
- said foam used in filling mode is allowed to drain.

12. Method according to any one of claims 7 to 11, **characterised in that**, following said contacting, either the foam is retrieved by suction before complete drying or the dry residue of this foam is retrieved by suction or by wiping.

FIG.1A

FIG.1B

FIG.2A

FIG.2B

Support

Mousse

Centrifugation +
retrait surnageant

1 heure en milieu **nutritif** à 30°C

Comptage

# FIG.3

FIG.4A

FIG.4B

FIG.5

FIG.6

FIG.7

FIG.8

FIG.9A

FIG.9B

FIG.10

FIG.11A

FIG.11B

FIG.11C

## RÉFÉRENCES CITÉES DANS LA DESCRIPTION

**Documents brevets cités dans la description**

- FR 2980367
- US 20070249509 A
- FR 2912668
- WO 0145505 A

- WO 2004008463 A
- WO 02043847 A
- US 7276468 B

**Littérature non-brevet citée dans la description**

- MATERIAL SAFETY DATA SHEET NAME OF FINISHED SOLUTION: EasyDECON® DF200-531X. EFT Holdings Inc, 2008
- Performance Data. EFT Holdings Inc, 2011
- *Allen Vanguard - CASCAD™ Decontamination Foam,* 2009, http://reports.hms-online.org/ViewProduct.aspx?CategoryId=175&ProductId=721

- Biological Agent Decontamination Technology Testing. *U.S. EPA. Biological Agent Decontamination Technology Testing . U.S. Environmental Protection Agency,* 2010
- A quantitative kinetic theory of emulsion type, I. Physical chemistry of the emulsifying agent. *Gas/Liquid and Liquid/Liquid Interface. Proceedings of the International Congress of Surface Activity,* 1957, 426-438